(19) Europäisches Patentamt
European Patent Office
Office européen des brevets

(11) **EP 4 497 771 A1**

(12) **EUROPEAN PATENT APPLICATION**
published in accordance with Art. 153(4) EPC

(43) Date of publication:
**29.01.2025 Bulletin 2025/05**

(21) Application number: **23774973.4**

(22) Date of filing: **22.03.2023**

(51) International Patent Classification (IPC):
***C08G 64/02*** *(2006.01)* ***C07D 317/44*** *(2006.01)*
***C07D 317/46*** *(2006.01)* ***C08G 64/30*** *(2006.01)*
***C08G 64/38*** *(2006.01)* ***C08L 69/00*** *(2006.01)*
***G02B 1/04*** *(2006.01)*

(52) Cooperative Patent Classification (CPC):
**C07D 317/44; C07D 317/46; C08G 64/02; C08G 64/30; C08G 64/38; C08L 69/00; G02B 1/04**

(86) International application number:
**PCT/JP2023/011315**

(87) International publication number:
**WO 2023/182378 (28.09.2023 Gazette 2023/39)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.03.2022 JP 2022046575**
**23.03.2022 JP 2022046565**

(71) Applicant: **Asahi Kasei Kabushiki Kaisha**
**Tokyo 1000006 (JP)**

(72) Inventors:
- **TODA, Tatsuro**
  **Tokyo 100-0006 (JP)**
- **NAKATA, Takuto**
  **Tokyo 100-0006 (JP)**
- **FUKUOKA, Hiroshi**
  **Tokyo 100-0006 (JP)**
- **YONEDA, Hisanari**
  **Tokyo 100-0006 (JP)**

(74) Representative: **D Young & Co LLP**
**3 Noble Street**
**London EC2V 7BQ (GB)**

(54) **POLYCARBONATE RESIN, POLYCARBONATE RESIN COMPOSITION, OPTICAL COMPONENT, AND PRODUCTION METHOD FOR POLYCARBONATE RESIN**

(57) A polycarbonate resin having a structural unit represented by the following formula (1) wherein A is an optionally substituted trivalent alicyclic moiety, and B is an optionally substituted monovalent alicyclic moiety, and a cyclic carbonate having a structure where an alicyclic skeleton is bonded to a 1,2-cycloalkylene carbonate skeleton via a single bond.

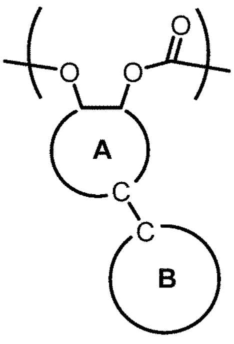

(1)

Figure 1

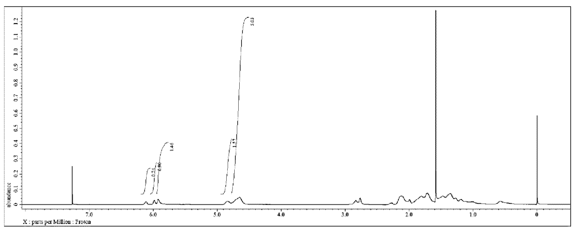

EP 4 497 771 A1

## Description

Technical Field

The present invention relates to a polycarbonate resin, a polycarbonate resin composition, an optical component, and a method for producing the polycarbonate resin.

Background Art

Polycarbonate resins are engineering plastics excellent in heat resistance and are required to possess excellent heat resistance and optical characteristics, in addition to their weight saving and lower costs. The development of specialty polycarbonate resins based on aromatic skeletons is actively underway for the purpose of satisfying such requirements.

On the other hand, a polycarbonate resin having an aliphatic, particularly, alicyclic structure, is also under development as a resin that is an alternative to aromatic polycarbonate. Alicyclic polycarbonate tends to be superior in light resistance and optical characteristics to polycarbonate resins having an aromatic ring such as bisphenol A. For example, Patent Literature 1 discloses a polycyclic alicyclic polycarbonate resin excellent in transparency, heat resistance, and color. Furthermore, polycarbonate is also under development using not only petroleum feedstocks but starting materials derived from biomasses such as plants. For example, Patent Literature 2 discloses a polycarbonate resin obtained using isosorbide inducible from starch as a starting material.

Among such alicyclic polycarbonate resins, poly(cyclohexene carbonate) having a cyclohexane carbonate structure is the simplest polycarbonate having a saturated six-membered carbon ring corresponding to a benzene ring. It is widely known that, as shown in, for example, Patent Literature 3, poly(cyclohexene carbonate) can be synthesized through the reaction of cyclohexene oxide with carbon dioxide. It is also known that, as described in Patent Literature 4 and Non Patent Literature 1, poly(cyclohexene carbonate) is obtained by the ring-opening polymerization of 1,2-cyclohexene carbonate.

Citation List

Patent Literature

Patent Literature 1: Japanese Patent No. 4774610
Patent Literature 2: Japanese Patent No. 6507495
Patent Literature 3: Japanese Patent No. 5403537
Patent Literature 4: Japanese Patent Laid-Open No. 2019-108547

Non Patent Literature

Non Patent Literature 1: Macromolecules 2014, 47, 4230-4235

Summary of Invention

Specifically, the present invention encompasses the following embodiments.

<1> A polycarbonate resin having a structural unit represented by the following formula (1):

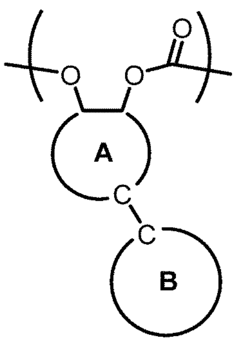

(1)

wherein A is an optionally substituted trivalent alicyclic moiety, and B is an optionally substituted monovalent alicyclic moiety.

<2> The polycarbonate resin according to <1>, wherein the structural unit represented by the formula (1) has a structural unit represented by the following formula (1A) :

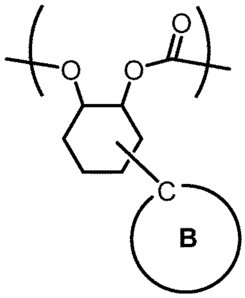

(1 A)

wherein B is an optionally substituted monovalent alicyclic moiety.

<3> The polycarbonate resin according to <1> or <2>, wherein the alicyclic moiety B is a group represented by the following formula (1a):

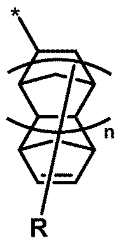

(1 a)

wherein R is a substituent, n is an integer of 0 to 2, and * is a binding site.

<4> The polycarbonate resin according to <1> or <2>, wherein the alicyclic moiety B is a group represented by the following formula (1b):

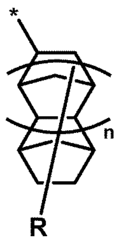

(1 b)

wherein R is a substituent, n is an integer of 0 to 2, and * is a binding site.

<5> The polycarbonate resin according to <1>, wherein the polycarbonate resin has a structural unit represented by the following formula (1B):

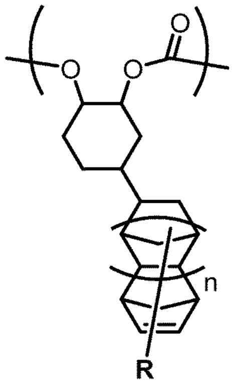

(1 B)

wherein R is a substituent, and n is an integer of 0 to 2.

<6> The polycarbonate resin according to <1>, wherein the polycarbonate resin has a structural unit represented by the following formula (1C):

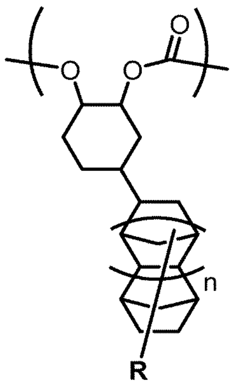

(1 C)

wherein R is a substituent, and n is an integer of 0 to 2.

<7> The polycarbonate resin according to <1>, wherein the polycarbonate resin has a structural unit represented by the following formula (1B-H):

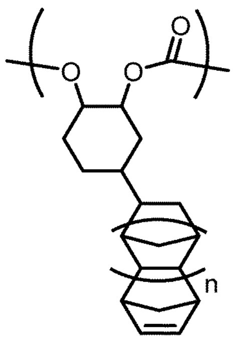

(1 B−H)

wherein n is an integer of 0 to 1.

<8> The polycarbonate resin according to <1>, wherein the polycarbonate resin has a structural unit represented by the following formula (1C-H):

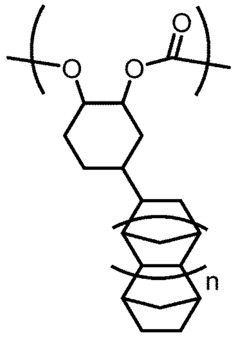

(1C−H)

wherein n is an integer of 0 to 1.

<9> The polycarbonate resin according to any of <1> to <8>, wherein the polycarbonate resin has a structural unit represented by the following formula (2):

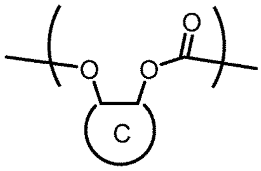

(2)

wherein C is an optionally substituted divalent alicyclic moiety.

<10> The polycarbonate resin according to any of <1> to <9>, wherein a weight-average molecular weight (Mw) is 10,000 or larger and 1,000,000 or smaller.

<11> The polycarbonate resin according to any of <1> to <10>, wherein a glass transition temperature (Tg) is 125°C or higher and 250°C or lower.

<12> The polycarbonate resin according to any of <1> to <11>, wherein an absolute value of a photoelastic coefficient is $1.5 \times 10^{-12}$ $Pa^{-1}$ or smaller.

<13> The polycarbonate resin according to any of <1> to <12>, wherein an absolute value of an in-plane phase difference in a 100% uniaxially stretched film of the polycarbonate resin is 100 nm or smaller in terms of a thickness of 100 μm.

<14> The polycarbonate resin according to any of <1> to <13>, wherein temperature dependence of orientation double refraction (dΔn/dT) in a stretched film obtained by uniaxial stretching under the following conditions satisfies $-0.1 \times 10^{-5} \leq d\Delta n/dT \leq +0.1 \times 10^{-5}$:

(stretching conditions)
stretching temperature: a temperature higher by 20°C than glass transition temperature Tg of the polycarbonate resin
stretching rate: 50 mm/min
stretch ratio: 100%

<15> A polycarbonate resin composition comprising the polycarbonate resin according to any of <1> to <14> and an antioxidant.

<16> An optical component comprising the polycarbonate resin according to any of <1> to <14>.

<17> Use of the polycarbonate resin according to any of <1> to <14> as a material for an optical component.

<18> A method for producing a polycarbonate resin having a structural unit represented by the following formula (1), comprising

a polymerization step of ring-opening polymerizing a cyclic carbonate represented by the following formula (1L) to obtain the polycarbonate resin:

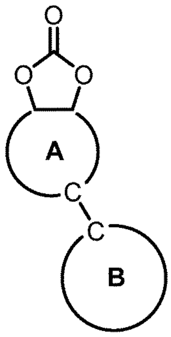

(1 L)

wherein A is an optionally substituted trivalent alicyclic moiety, and B is an optionally substituted monovalent alicyclic moiety,

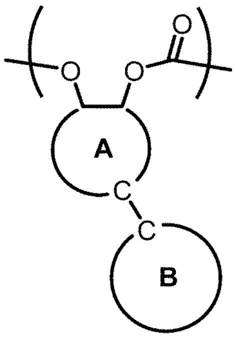

(1)

wherein A is an optionally substituted trivalent alicyclic moiety, and B is an optionally substituted monovalent alicyclic moiety.

<19> A cyclic carbonate having a structure where an alicyclic skeleton is bonded to a 1,2-cycloalkylene carbonate skeleton via a single bond.

<20> The cyclic carbonate according to <19>, wherein the cyclic carbonate is represented by the following formula (1L) :

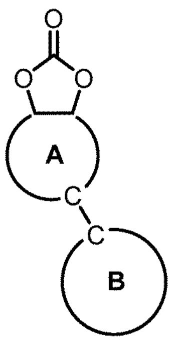

(1 L)

wherein A is an optionally substituted trivalent alicyclic moiety, and B is an optionally substituted monovalent alicyclic moiety.

<21> The cyclic carbonate according to <20>, wherein the formula (1L) is the following formula (1LA):

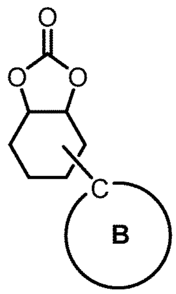

(1 L A)

wherein B is an optionally substituted monovalent alicyclic moiety.

<22> The cyclic carbonate according to <20> or <21>, wherein the alicyclic moiety B is a group represented by the following formula (1a):

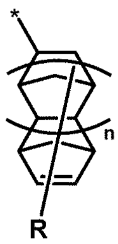

(1 a)

wherein R is a substituent, n is an integer of 0 to 2, and * is a binding site.

<23> The cyclic carbonate according to <20> or <21>, wherein the alicyclic moiety B is a group represented by the following formula (1b):

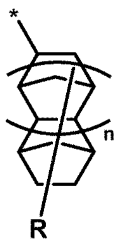

(1 b)

wherein R is a substituent, n is an integer of 0 to 2, and * is a binding site.

<24> The cyclic carbonate according to <20> or <21>, wherein the alicyclic moiety B is an optionally substituted norbornyl group, an optionally substituted norbornenyl group, an optionally substituted decahydro-1,4:5,8-dimethanonaphthalenyl group, or an optionally substituted octahydro-1,4:5,8-dimethanonaphthalenyl group.

<25> The cyclic carbonate according to <19>, wherein the cyclic carbonate is represented by the following formula (1LB) :

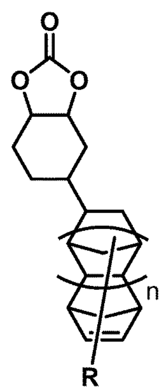

(1 L B)

wherein R is a substituent, and n is an integer of 0 to 2.

<26> The cyclic carbonate according to <19>, wherein the cyclic carbonate is represented by the following formula (1LC) :

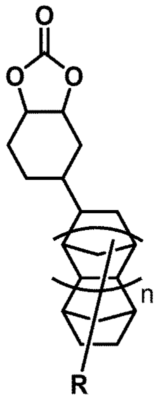

(1 L C)

wherein R is a substituent, and n is an integer of 0 to 2.

<27> The cyclic carbonate according to <19>, wherein the cyclic carbonate is represented by

the following formula (1L-1):

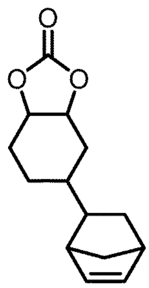

(1 L－1)

the following formula (1L-2):

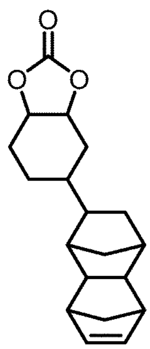

(1 L－2)

the following formula (1L-3):

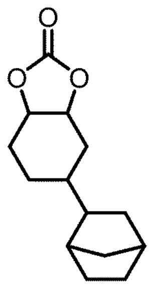

(1 L－3)

or the following formula (1L-4):

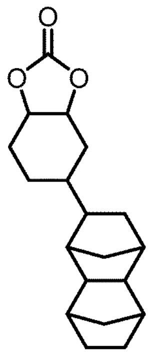

(1 L－4)

<28> A method for producing a cyclic carbonate having a structure where an alicyclic skeleton is bonded to a 1,2-cycloalkylene carbonate skeleton via a single bond, comprising the step of reacting a cyclic carbonate represented by the following formula (2L) with a compound having a conjugated diene moiety:

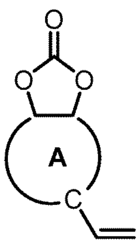

(2 L)

wherein A is an optionally substituted trivalent alicyclic moiety.

<29> A cyclic carbonate composition comprising

a cyclic carbonate represented by the following formula (1L-1):

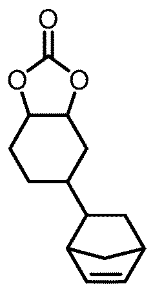

(1 L－1)

and a cyclic carbonate represented by the following formula (1L-2):

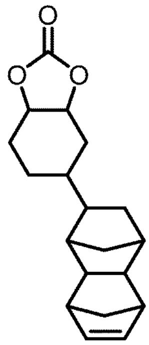
(1 L－2)

<30> A cyclic carbonate composition comprising

a cyclic carbonate represented by the following formula (1L-3):

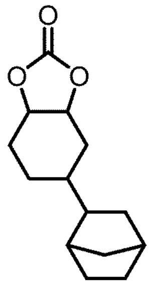
(1 L－3)

and a cyclic carbonate represented by the following formula (1L-4):

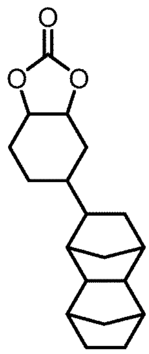
(1 L－4)

Advantageous Effects of Invention

The present invention can provide a polycarbonate resin having excellent heat resistance and having a low photoelastic coefficient, a polycarbonate resin composition comprising the same, a method for producing the polycarbonate resin, etc.

Brief Description of Drawings

[Figure 1] Figure 1 shows a $^{1}$H-NMR spectrum of a polycarbonate resin in Example 1.
[Figure 2] Figure 2 shows a $^{1}$H-NMR spectrum of a polycarbonate resin in Example 2.
[Figure 3] Figure 3 shows a $^{1}$H-NMR spectrum of a polycarbonate resin in Example 3.
[Figure 4] Figure 4 shows a $^{1}$H-NMR spectrum of a polycarbonate resin in Example 4.
[Figure 5] Figure 5 shows a $^{1}$H-NMR spectrum of a polycarbonate resin in Example 5.
[Figure 6] Figure 6 shows a $^{1}$H-NMR spectrum of a polycarbonate resin in Example 6.

[Figure 7] Figure 7 shows a $^1$H-NMR spectrum of a polycarbonate resin in Example 7.
[Figure 8] Figure 8 shows a $^1$H-NMR spectrum of a polycarbonate resin in Example 8.
[Figure 9] Figure 9 shows a $^1$H-NMR spectrum of a cyclic carbonate (TCDT) in Example L1.
[Figure 10] Figure 10 shows a $^1$H-NMR spectrum of a cyclic carbonate (NORT) in Example L2.
[Figure 11] Figure 11 shows a $^1$H-NMR spectrum of a cyclic carbonate (NORT-H) in Example L6.
[Figure 12] Figure 12 shows a $^1$H-NMR spectrum of a cyclic carbonate (TCDT-H) in Example L7.
[Figure 13] Figure 13 shows a $^1$H-NMR spectrum of a cyclic carbonate ($Me_5$NORT) in Example L11.

Description of Embodiments

Hereinafter, the mode for carrying out the present invention (hereinafter, also referred to as the "present embodiment") will be described in detail. However, the present invention is not limited by the present embodiment and can be carried out by making various changes or modifications without departing from the gist of the present invention.

[Alicyclic polycarbonate resin]

The present inventors have conducted detailed studies on conventional alicyclic polycarbonate resins including those described in the literatures, and consequently found that any of heat resistance and optical characteristics are insufficient.

As described in, for example, Patent Literature 4 and Non Patent Literature 1, a polycarbonate resin having a cyclic cyclohexane structure in the backbone has a glass transition temperature higher than that of aliphatic chain polycarbonate but lower than that of a general-purpose aromatic polycarbonate resin having a bisphenol A structure, and thus still has a challenge from the viewpoint of heat resistance such as morphological stability in a high-temperature environment.

Poly(cyclohexene carbonate) has a challenge to a lower double refraction, though its photoelastic coefficient serving as a control factor for a double refraction that occurs in use is low and offers low birefringence.

An object of the present invention is to provide a polycarbonate resin having excellent heat resistance and having a low photoelastic coefficient, a polycarbonate resin composition comprising the same, a method for producing the polycarbonate resin, etc.

The present inventors have pursued diligent studies to attain the object and consequently found that a polycarbonate resin having a specific cyclic structure exhibits excellent heat resistance and exhibits a low photoelastic coefficient.

The polycarbonate resin of the present embodiment has a structural unit represented by the following formula (1):

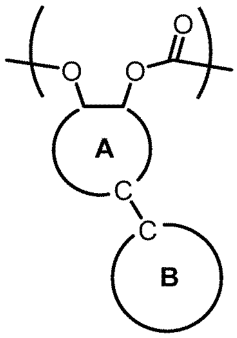
(1)

wherein A is an optionally substituted trivalent alicyclic moiety, and B is an optionally substituted monovalent alicyclic moiety.

The polycarbonate resin according to the present embodiment has excellent heat resistance and has a low photoelastic coefficient. The reason for this is probably, but is not limited to, as follows: most of polycarbonate resins having a conventional alicyclic structure are 1,2-cycloalkylene carbonate having no ring skeleton in a side chain and thus have insufficient heat resistance. In addition, their photoelastic coefficients cannot be sufficiently reduced. By contrast, the polycarbonate resin of the present embodiment has a structure where an alicyclic skeleton is further bonded to a side chain of a 1,2-cycloalkylene carbonate skeleton. Therefore, its heat resistance is presumably improved with increase in the proportion of the ring skeleton in a molecule. Furthermore, the flatness of the whole molecule is reduced with increase in the proportion of the alicyclic skeleton in the molecule, presumably exerting a low photoelastic coefficient.

The structural unit represented by the formula (1) is preferably the following trans form.

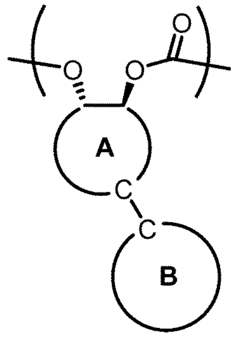

The above drawing illustrates a conformation, and if the formula involves a chiral center, any of R and S conformations may be used.

In the formula (1), A is an optionally substituted trivalent alicyclic moiety. A corresponds to a cycloalkane-1,2-diyl group. In the formula (1), examples of A include a cyclopropane-1,2-diyl group, a cyclobutane-1,2-diyl group, a cyclopentane-1,2-diyl group, a cyclohexane-1,2-diyl group, a cycloheptane-1,2-diyl group, a cyclooctane-1,2-diyl group, a cyclononane-1,2-diyl group, and a cyclodecane-1,2-diyl group. Among them, A is preferably a cyclobutane-1,2-diyl group, a cyclopentane-1,2-diyl group, a cyclohexane-1,2-diyl group, a cycloheptane-1,2-diyl group, or a cyclooctane-1,2-diyl group, more preferably a cyclobutane-1,2-diyl group, a cyclopentane-1,2-diyl group, a cyclohexane-1,2-diyl group, or a cycloheptane-1,2-diyl group.

In the formula (1), B is an optionally substituted alicyclic skeleton. Examples of the alicyclic skeleton B include, but are not particularly limited to, cyclopropane, cyclopropene, cyclobutane, cyclobutene, cyclopentane, cyclopentene, cyclohexane, cyclohexene, cycloheptane, cycloheptene, cyclooctane, cyclooctene, cyclononane, cyclononene, cyclodecane, cyclodecene, groups represented by the following formula (1a), and groups represented by the following formula (1b):

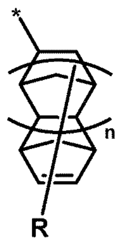

(1 a)

wherein R is a substituent, n is an integer of 0 to 2, and * is a binding site,

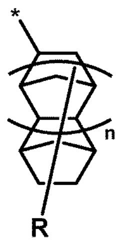

(1 b)

wherein R is a substituent, n is an integer of 0 to 2, and * is a binding site.

Examples of the group represented by the formula (1a) or the group represented by the formula (1b) include, but are not particularly limited to, norbornane, norbornene, adamantane, bicyclo[2.2.2]octane, bicyclo[2.2.2]octene, decahydronaphthalene, decahydro-1,4:5,8-dimethanonaphthalene, and octahydro-1,4:5,8-dimethanonaphthalene.

In the formula (1), B is preferably cyclopropane, cyclopropene, cyclobutane, cyclobutene, cyclopentane, cyclopentene, cyclohexane, cyclohexene, cycloheptane, cycloheptene, norbornane, norbornene, adamantane, bicyclo[2.2.2]octane, bicyclo[2.2.2]octene, decahydronaphthalene, decahydro-1,4:5,8-dimethanonaphthalene, or octahydro-1,4:5,8-dimethanonaphthalene. From a similar viewpoint, in the formula (1), B is more preferably cyclopropane, cyclopropene, cyclobutane, cyclobutene, cyclopentane, cyclopentene, cyclohexane, cyclohexene, cycloheptane, cycloheptene, norbornane, norbornene, adamantane, decahydronaphthalene, decahydro-1,4:5,8-dimethanonaphthalene, or octahydro-1,4:5,8-dimethanonaphthalene. From a similar viewpoint, in the formula (1), B is further preferably cyclopropane, cyclopropene, cyclobutane, cyclobutene, cyclopentane, cyclopentene, cyclohexane, cyclohexene, cycloheptane, cycloheptene, norbornane, norbornene, decahydronaphthalene, decahydro-1,4:5,8-dimethanonaphthalene, or octahydro-1,4:5,8-dimethanonaphthalene.

The alicyclic moiety B is preferably a group represented by the formula (1a) or a group represented by the formula (1b), more preferably an optionally substituted norbornyl group, an optionally substituted norbornenyl group, an optionally substituted decahydro-1,4:5,8-dimethanonaphthalenyl group, or an optionally substituted octahydro-1,4:5,8-dimethanonaphthalenyl group, further preferably a norbornyl group, a norbornenyl group, a decahydro-1,4:5,8-dimethanonaphthalenyl group, or an octahydro-1,4:5,8-dimethanonaphthalenyl group, from the viewpoint of improving heat resistance.

In the specification of the present application, when each group is substituted, the substituent is not particularly limited and is, for example, a hydroxy group, a phosphoric acid group, an aryl group having 6 to 20 carbon atoms, an aralkyl group having 6 to 20 carbon atoms, an alkoxy group having 1 to 20 carbon atoms, a silyl group having 1 to 30 carbon atoms, a silylalkoxy group having 1 to 30 carbon atoms, an ester group having 1 to 11 carbon atoms, an acyl group having 1 to 11 carbon atoms, or an unsubstituted linear, branched, or cyclic alkyl group having 1 to 30 carbon atoms. In the present embodiment, each of A and B may have a plurality of substituents. In this case, the substituents may be the same as or different from each other.

In the present embodiment, in the formula (1), the substituent is a hydroxy group, a phosphoric acid group, an aryl group having 6 to 20 carbon atoms, an aralkyl group having 6 to 20 carbon atoms, an alkoxy group having 1 to 20 carbon atoms, a silyl group having 1 to 30 carbon atoms, a silylalkoxy group having 1 to 30 carbon atoms, an ester group having 1 to 11 carbon atoms, an acyl group having 1 to 11 carbon atoms, or an unsubstituted linear, branched, or cyclic alkyl group having 1 to 30 carbon atoms. In the formula (1), the substituent is preferably one or more substituents selected from the group consisting of a hydroxy group, an alkoxy group having 1 to 20 carbon atoms, an ester group having 1 to 11 carbon atoms, an acyl group having 1 to 11 carbon atoms, and an unsubstituted linear, branched, or cyclic alkyl group having 1 to 30 carbon atoms. In the formula (1), the substituent is more preferably one or more substituents selected from the group consisting of a hydroxy group, an alkoxy group having 1 to 20 carbon atoms, and an unsubstituted linear, branched, or cyclic alkyl group having 1 to 30 carbon atoms. In the formula (1), the substituent is further preferably one or more substituents selected from the group consisting of an alkoxy group having 1 to 20 carbon atoms, and an unsubstituted linear, branched, or cyclic alkyl group having 1 to 30 carbon atoms.

The phosphoric acid group mentioned above may be unsubstituted or substituted. Specifically, this group may be a monosubstituted phosphoric acid group or may be a disubstituted phosphoric acid group. When the phosphoric acid group is substituted, the substituent is preferably an unsubstituted linear, branched, or cyclic alkyl group having 1 to 30 carbon atoms from the viewpoint of more effectively and reliably exerting the advantageous effects of the present invention. From a similar viewpoint, the phosphoric acid group according to the present embodiment is preferably unsubstituted.

Examples of the aryl group having 6 to 20 carbon atoms mentioned above include, but are not particularly limited to: unsubstituted aryl groups or aryl groups having an alkyl group, such as a phenyl group, a methylphenyl group, a dimethylphenyl group, a trimethylphenyl group, a tetramethylphenyl group, a pentamethylphenyl group, an ethylphenyl group, a propylphenyl group, and a diisopropylphenyl group; aryl groups having an alkoxy group, such as a 4-methoxyphenyl group and a 3,5-dimethoxyphenyl group; and a biphenyl group, a naphthyl group, and an anthracenyl group.

Examples of the aralkyl group having 6 to 20 carbon atoms mentioned above include, but are not particularly limited to: unsubstituted aralkyl groups or aralkyl groups having an alkyl group, such as a benzyl group, a 4-methylbenzyl group, and a phenethyl group; aralkyl groups having an alkoxy group, such as a 4-methoxybenzyl group and a 3,5-dimethoxybenzyl group; and a diphenylmethyl group, a naphthylmethyl group, and an anthracenylmethyl group.

Examples of the alkoxy group having 1 to 20 carbon atoms mentioned above include, but are not particularly limited to, a methoxy group, an ethoxy group, a propoxy group, a butoxy group, a pentyloxy group, a cyclopentyloxy group, a hexyloxy group, a cyclohexyloxy group, a heptyloxy group, an octyloxy group, a nonanyloxy group, a decyloxy group, a phenoxy group, a benzyloxy group, a vinyloxy group, and an allyloxy group.

Examples of the silyl group having 1 to 30 carbon atoms mentioned above include, but are not particularly limited to, a trimethylsilyl group, a triethylsilyl group, a triisopropylsilyl group, a triphenylsilyl group, a tert-butyldimethylsilyl group, a di-tert-butylisobutylsilyl group, and a tert-butyldiphenylsilyl group.

Examples of the silylalkoxy group having 1 to 30 carbon atoms mentioned above include, but are not particularly limited to, a trimethylsilylmethoxy group, a trimethylsilylethoxy group, a trimethylsilylphenoxy group, a trimethylsilylbenzyloxy group, a triethylsilylmethoxy group, a triethylsilylethoxy group, a triethylsilylphenoxy group, a triethylsilylbenzyloxy group, a triisopropylsilylmethoxy group, a triisopropylsilylethoxy group, a triisopropylsilylphenoxy group, a triisopropylsilylbenzyloxy group, a triphenylsilylmethoxy group, a triphenylsilylethoxy group, a triphenylsilylphenoxy group, a triphenylsilylbenzyloxy group, a tert-butyldimethylsilylmethoxy group, a tert-butyldimethylsilylethoxy group, a tert-butyldimethylsilylphenoxy group, a tert-butyldimethylsilylbenzyloxy group, a di-tert-butylisobutylsilylmethoxy group, a di-tert-butylisobutylsilylethoxy group, a di-tert-butylisobutylsilylphenoxy group, a di-tert-butylisobutylsilylbenzyloxy group, a tert-butyldiphenylsilylmethoxy group, a tert-butyldiphenylsilylethoxy group, a tert-butyldiphenylsilylphenoxy group, and a tert-butyldiphenylsilylbenzyloxy group.

Examples of the ester group having 1 to 11 carbon atoms mentioned above include, but are not particularly limited to a methyl ester group, an ethyl ester group, a propyl ester group, a butyl ester group, a pentyl ester group, a cyclopentyl ester group, a hexyl ester group, a cyclohexyl ester group, a heptyl ester group, an octyl ester group, a nonanyl ester group, a decyl ester group, a phenyl ester group, a benzyl ester group, a vinyl ester group, and an allyl ester group.

Examples of the acyl group having 1 to 11 carbon atoms mentioned above include, but are not particularly limited to, a formyl group, an acetyl group, a propionyl group, a butyryl group, a valeryl group, and a benzoyl group.

Examples of the unsubstituted linear, branched, or cyclic alkyl group having 1 to 30 carbon atoms mentioned above include a methyl group, an ethyl group, a n-propyl group, an isopropyl group, a n-butyl group, an isobutyl group, a tert-butyl group, a n-pentyl group, a cyclopentyl group, a n-hexyl group, a cyclohexyl group, a n-heptyl group, a 1-norbornyl group, a 2-norbornyl group, a n-octyl group, a 1-bicyclo[2.2.2]octyl group, a 2-bicyclo[2.2.2]octyl group, a n-nonanyl group, a n-decyl group, a 1-adamantyl group, a 2-adamantyl group, a decahydronaphthyl group, and a tetracyclododecyl group.

(Terminal structure)

A terminal group of the polycarbonate resin of the present embodiment is not particularly limited and is, for example, a hydrogen atom, a hydroxy group, a phosphoric acid group, an amino group, an aryl group having 6 to 20 carbon atoms, an aralkyl group having 6 to 20 carbon atoms, an alkoxy group having 1 to 20 carbon atoms, a silyl group having 1 to 30 carbon atoms, a silylalkoxy group having 1 to 30 carbon atoms, an ester group having 1 to 11 carbon atoms, an acyl group having 1 to 11 carbon atoms, or an unsubstituted linear, branched, or cyclic alkyl group having 1 to 30 carbon atoms. The polycarbonate resin of the present embodiment may form a cyclic structure through both ends boned to each other. Specifically, the polycarbonate resin of the present embodiment may have no terminal structure. The polycarbonate resin of the present embodiment preferably has hydroxy groups at both ends.

The polycarbonate resin of the present embodiment preferably has a structure represented by the following formula (1A):

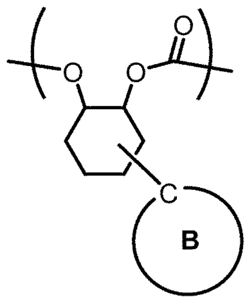

(1 A)

wherein B is an optionally substituted monovalent alicyclic moiety.

Preferred B in the formula (1A) is as defined in the formula (1). In the formula (1A), when each group is substituted, examples of the substituent are also as defined in the formula (1).

The polycarbonate resin of the present embodiment preferably has a structural unit represented by the following formula (1B) from the viewpoint of facilitating introducing an additional substituent:

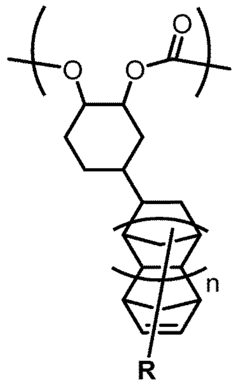

(1 B)

wherein R is a substituent, and n is an integer of 0 to 2.

The polycarbonate resin of the present embodiment preferably has a structural unit represented by the following formula (1C) from the viewpoint of more improving heat resistance:

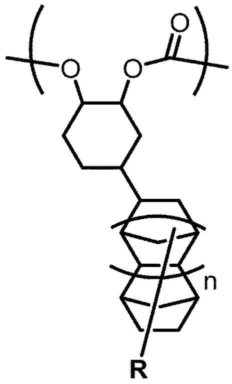

(1 C)

wherein R is a substituent, and n is an integer of 0 to 2.

In the formulas (1B) and (1C), n is preferably 0 or 1.

The polycarbonate resin of the present embodiment preferably has a structural unit represented by the following formula (1B-H) from the viewpoint of facilitating introducing an additional substituent and obtaining a lower photoelastic coefficient:

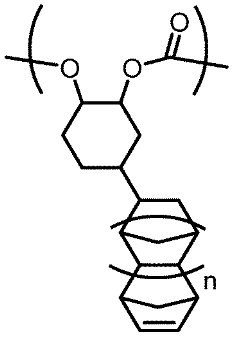

(1 B－H)

wherein n is an integer of 0 to 1.

The polycarbonate resin of the present embodiment preferably has a structural unit represented by the following formula (1C-H) from the viewpoint of facilitating introducing an additional substituent and obtaining a lower photoelastic coefficient:

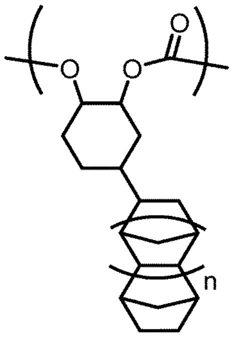

(1 C－H)

wherein n is an integer of 0 to 1.

The cyclic carbonate having the structural unit represented by the formula (1B) preferably has a structural unit represented by the following formula (1B-1) or a structural unit represented by the following formula (1B-2) from the viewpoint of facilitating introducing an additional substituent and obtaining a lower photoelastic coefficient:

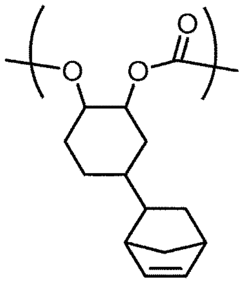

(1 B－1)

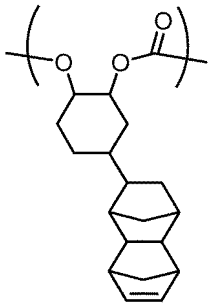

(1 B－2)

The cyclic carbonate having the structural unit represented by the formula (1C) preferably has a structural unit represented by the following formula (1C-1) or a structural unit represented by the following formula (1C-2) from the viewpoint of more improving heat resistance and obtaining a lower photoelastic coefficient:

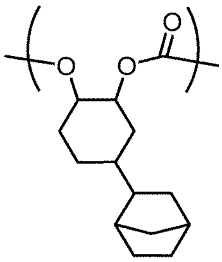

(1 C－1)

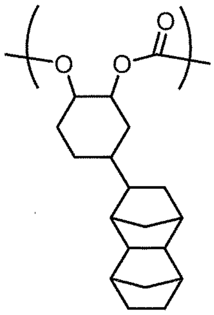

(1 C－2)

The polycarbonate resin of the present embodiment may comprise two or more types of structural units represented by the formula (1).

The polycarbonate resin of the present embodiment may have an additional structural unit, in addition to the structural unit represented by the formula (1).

Examples of the additional structural unit include ring-opened cyclic carbonate units.

More specifically, the polycarbonate resin of the present embodiment preferably has, as the additional structural unit, a structural unit represented by the following formula (2):

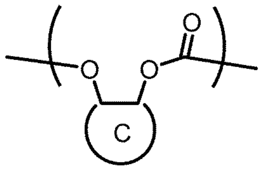

(2)

wherein C is an optionally substituted divalent alicyclic moiety.

Examples of the alicyclic moiety C include a cyclopropane-1,2-diyl group, a cyclobutane-1,2-diyl group, a cyclopentane-1,2-diyl group, a cyclohexane-1,2-diyl group, a cycloheptane-1,2-diyl group, a cyclooctane-1,2-diyl group, a cyclononane-1,2-diyl group, and a cyclodecane-1,2-diyl group. Among them, C is preferably a cyclobutane-1,2-diyl group, a cyclopentane-1,2-diyl group, a cyclohexane-1,2-diyl group, a cycloheptane-1,2-diyl group, or a cyclooctane-1,2-diyl group, more preferably a cyclobutane-1,2-diyl group, a cyclopentane-1,2-diyl group, a cyclohexane-1,2-diyl group, or a cycloheptane-1,2-diyl group.

When each group is substituted, examples of the substituent are as defined in the formula (1).

The proportion of the structural unit represented by the formula (1) in the polycarbonate resin of the present embodiment is not particularly limited and is preferably, for example, 10 to 90 mol%, 20 to 80 mol%, 30 to 70 mol%, or 40 to 60 mol%.

The proportion of the structural unit represented by the formula (2) in the polycarbonate resin of the present embodiment is not particularly limited and is preferably, for example, 10 to 90 mol%, 20 to 80 mol%, 30 to 70 mol%, or 40 to 60 mol%.

In the polycarbonate resin of the present embodiment, the weight-average molecular weight (Mw) is preferably 10,000 or larger and 1,000,000 or smaller. The polycarbonate resin of the present embodiment having the Mw that falls within the range described above is easy to mold. Such a polycarbonate resin is excellent in heat resistance. From a similar viewpoint, the Mw is more preferably 10,000 or larger and 950,000 or smaller, further preferably 10,000 or larger and 900,000 or smaller. The upper limit of the Mw may be 800,000. The weight-average molecular weight is a polystyrene-based value measured by gel permeation chromatography and can be measured, specifically, by a method described in Examples.

In order to control the weight-average molecular weight (Mw) of the polycarbonate resin of the present embodiment within the range described above, the ratio between a polymerizable monomer and a polymerization initiator can be appropriately adjusted, and the polycarbonate resin can be produced by a production method mentioned later. Polymerization reaction progresses at a favorable rate of conversion and the Mw tends to be able to be increased, by controlling the amount of the polymerization initiator added to the polymerizable monomer within a specific range.

In the polycarbonate resin of the present embodiment, the glass transition temperature (Tg) is preferably 125°C or higher and 250°C or lower. When the Tg is 125°C or higher, morphology tends to be able to be further maintained even in an environment of usage having a relatively high temperature. Thus, heat resistance is excellent. When the Tg is 250°C or lower, molding processability tends to be much better. From a similar viewpoint, the glass transition temperature of the polycarbonate resin of the present embodiment is more preferably 125°C or higher and 245°C or lower, further preferably 125°C or higher and 240°C or lower. The glass transition temperature of the polycarbonate resin is measured with a differential scanning calorimeter (DSC) and can be measured, specifically, by a method described in Examples.

In order to control the glass transition temperature of the polycarbonate resin of the present embodiment within the preferred range described above, A, B, and R in the formula (1) can be appropriately selected. Particularly, when A is a cyclohexane-1,2-diyl group, B is a cyclohexyl group, a norbornyl group, a decahydronaphthyl group, or an octahydro-1,4:5,8-dimethanonaphthalenyl group, and a hydrogen atom, an alkoxy group having 1 to 20 carbon atoms, or an unsubstituted linear, branched, or cyclic alkyl group having 1 to 30 carbon atoms is selected as R, the glass transition temperature is improved and heat resistance tends to be excellent.

In the polycarbonate resin of the present embodiment, the absolute value of a photoelastic coefficient is preferably $1.5 \times 10^{-12}$ $Pa^{-1}$ or smaller. When the absolute value of the photoelastic coefficient falls within the range described above, change in phase difference can be suppressed when strain is applied to the polycarbonate resin. The polycarbonate resin therefore tends to exhibit lower birefringence. From a similar viewpoint, the absolute value of the photoelastic coefficient of the polycarbonate resin of the present embodiment is more preferably $1.4 \times 10^{-12}$ $Pa^{-1}$ or smaller, further preferably $1.3 \times 10^{-12}$ $Pa^{-1}$ or smaller, still further preferably $1.2 \times 10^{-12}$ $Pa^{-1}$ or smaller, even further preferably $1.1 \times 10^{-12}$ $Pa^{-1}$ or smaller. In the polycarbonate resin of the present embodiment, the lower limit of the absolute value of the photoelastic coefficient is not particularly limited and is, for example, $0.01 \times 10^{-12}$ $Pa^{-1}$. The photoelastic coefficient of the polycarbonate resin can be measured, specifically, by a method described in Examples. A test piece for use in measuring the photoelastic coefficient of the polycarbonate resin of the present embodiment is molded into a film at 150°C or higher and 300°C or lower. The film thickness of the prepared film is preferably 10 μm or larger and 1000 μm or smaller.

In order to control the absolute value of the photoelastic coefficient of the polycarbonate resin of the present embodiment within the preferred range described above, A, B, and R in the formula (1) can be appropriately selected. Particularly, when A is a cyclohexane-1,2-diyl group, B is a cyclohexyl group, a norbornyl group, a decahydronaphthyl group, or an octahydro-1,4:5,8-dimethanonaphthalenyl group, and a hydrogen atom, an alkoxy group having 1 to 20 carbon atoms, or an unsubstituted linear, branched, or cyclic alkyl group having 1 to 30 carbon atoms is selected as R, the absolute value of the photoelastic coefficient tends to be decreased.

In the polycarbonate resin of the present embodiment, the absolute value of an in-plane phase difference in a 100% uniaxially stretched film is preferably 100 nm or smaller in terms of a thickness of 100 μm. When the absolute value of the in-plane phase difference falls within the range described above, the double refraction of the polycarbonate resin ascribable to the orientation of molecules contained in the polymer chain tends to be able to be further prevented. From a similar viewpoint, the absolute value of the in-plane phase difference in the 100% uniaxially stretched film of the polycarbonate resin of the present embodiment is more preferably 95 nm or smaller in terms of a thickness of 100 μm, further preferably 90 nm or smaller in terms of a thickness of 100 μm. The lower limit of the absolute value of the in-plane phase difference is not particularly limited and may be 0 nm in terms of a thickness of 100 μm. The in-plane phase difference of the polycarbonate resin can be measured, specifically, by a method described in Examples. The 100% uniaxially stretched film of the polycarbonate resin means a film obtained by molding the polycarbonate resin into a film by a method such as a casting method or vacuum heat press, and then stretching the film by 100% in the uniaxial direction under a temperature condition equal to or higher than the glass transition temperature. The film before stretching is formed at 150°C or higher and 300°C or lower. The film thickness of the prepared film is preferably 10 μm or larger and 1000 μm or smaller.

In order to control the absolute value of the in-plane phase difference of the polycarbonate resin of the present embodiment within the preferred range described above, A, B, and R in the formula (1) can be appropriately selected. Particularly, when A is a cyclohexane-1,2-diyl group, B is a cyclohexyl group, a norbornyl group, a decahydronaphthyl group, or an octahydro-1,4:5,8-dimethanonaphthalenyl group, and a hydrogen atom, an alkoxy group having 1 to 20 carbon atoms, or an unsubstituted linear, branched, or cyclic alkyl group having 1 to 30 carbon atoms is selected as R, the absolute value of the in-plane phase difference tends to be decreased.

In the polycarbonate resin of the present embodiment, the temperature dependence of orientation double refraction (dΔn/dT) in a stretched film obtained by uniaxial stretching under conditions given below is preferably $-0.1 \times 10^{-5} \leq d\Delta n/dT \leq +0.1 \times 10^{-5}$. When the dΔn/dT falls within the range described above, the occurrence of a double refraction in the polycarbonate resin due to temperature change tends to be able to be further suppressed. Specifically, a uniaxially stretched film having a film thickness of 100 μm causes 1 nm change in phase difference by temperature change of 10°C at $d\Delta n/dT = 0.1 \times 10^{-5}$. This change is generally a level viewable by humans in crossed nicol observation. Therefore, the absolute value of the temperature dependence of orientation double refraction (dΔn/dT) is preferably $0.1 \times 10^{-5}$ or smaller. From a similar viewpoint, dΔn/dT of the polycarbonate resin of the present embodiment is more preferably $-0.05 \times 10^{-5} \leq d\Delta n/dT \leq +0.05 \times 10^{-5}$, further preferably $-0.01 \times 10^{-5} \leq d\Delta n/dT \leq +0.01 \times 10^{-5}$. The temperature dependence of orientation double refraction in a stretched film obtained by uniaxially stretching the polycarbonate resin under conditions given below can be measured, specifically, by a method described in Examples. The 100% uniaxially stretched film of the polycarbonate resin means a film obtained by molding the polycarbonate resin into a film by a method such as a casting method or vacuum heat press, and then stretching the film by 100% in the uniaxial direction under a temperature condition equal to or higher than the glass transition temperature. The film before stretching is formed at 150°C or higher and 300°C or lower. The film thickness of the prepared film is preferably 10 μm or larger and 1000 μm or smaller.

(Stretching conditions)

Stretching temperature: a temperature higher by 20°C than the glass transition temperature Tg of the polycarbonate resin
Stretching rate: 50 mm/min
Stretch ratio: 100%

In order to control, dΔn/dT of the polycarbonate resin of the present embodiment within the preferred range described above, A, B, and R in the formula (1) can be appropriately selected. Particularly, when A is a cyclohexane-1,2-diyl group, B is a cyclohexyl group, a norbornyl group, a decahydronaphthyl group, or an octahydro-1,4:5,8-dimethanonaphthalenyl group, and a hydrogen atom, an alkoxy group having 1 to 20 carbon atoms, or an unsubstituted linear, branched, or cyclic alkyl group having 1 to 30 carbon atoms is selected as R, dΔn/dT tends to be decreased.

[Polycarbonate resin composition]

The polycarbonate resin composition of the present embodiment contains the polycarbonate resin described above and an antioxidant.

The polycarbonate resin composition of the present embodiment containing an antioxidant can be suppressed from being degraded by heat or shear at the time of a molding process, and can therefore have more improved heat resistance. Furthermore, the polycarbonate resin composition of the present embodiment containing an antioxidant can prevent the polycarbonate resin from being oxidized in use, and can therefore have more improved light resistance.

Examples of the antioxidant in the polycarbonate resin composition of the present embodiment include, but are not particularly limited to, hindered phenol antioxidants and phosphorus antioxidants.

Examples of the hindered phenol antioxidant include, but are not particularly limited to, Irganox 1010 (pentaerythritol tetrakis[3-(3,5-di-t-butyl-4-hydroxyphenyl)propionate]), Irganox 1076 (octadecyl-3-(3,5-di-t-butyl-4-hydroxyphenyl)propionate), Irganox 1330 (3,3',3'',5,5',5''-hexa-t-butyl-a,a',a''-(mesitylene-2,4,6-triyl)tri-p-cresol), Irganox 3114 (1,3,5-tris(3,5-di-t-butyl-4-hydroxybenzyl)-1,3,5-triazine-2,4,6(1H,3H,5H)-trione), Irganox 3125, ADKSTAB AO-60 (pentaerythritoltetrakis[3-(3,5-di-t-butyl-4-hydroxyphenyl)propionate]), ADKSTAB AO-80 (3,9-bis{2-[3-(3-t-butyl-4-hydroxy-5-methylphenyl)propionyloxyJ-1,1-dimethylethyl}-2,4,8,10-tetraoxaspiro[5.5]undecane), Cyanox 1790, Sumilizer GA-80, Sumilizer GS (2-[1-(2-hydroxy-3,5-di-tert-pentylphenyl)ethyl]-4,6-di-tert-pentylphenyl acrylate), and Sumilizer GM (2-tert-butyl-4-methyl-6-(2-hydroxy-3-tert-butyl-5-methylbenzyl)phenyl acrylate). These may be used singly, or in combinations of two or more thereof.

Examples of the phosphorus antioxidant include, but are not particularly limited to, Irgafos 168 (tris(2,4-di-t-butylphenyl)phosphite), Irgafos 12 (tris[2-[[2,4,8,10-tetra-t-butyldibenzo[d,f][1,3,2]dioxaphosphepin-6-yl]oxy]ethyl] amine), ADKSTAB HP-10 (2,2'-methylenebis(4,6-di-tert-butylphenyl)octyl phosphite), ADKSTAB PEP36 (bis(2,6-di-t-butyl-4-methylphenyl)pentaerythritol diphosphite), ADKSTAB PEP36A (bis(2,6-di-t-butyl-4-methylphenyl)pentaerythritol diphosphite), Sumilizer GP ((6-[3-(3-t-butyl-4-hydroxy-5-methylphenyl)propoxy]-2,4,8,10-tetra-t-butyldibenzo[d,f][1,3,2] dioxaphosphepine), and GSY P101 (tetrakis(2,4-di-t-butyl-5-methylphenyl)4,4'-biphenylene diphosphonite). These may be used singly, or in combinations of two or more thereof.

The polycarbonate resin in the polycarbonate resin composition of the present embodiment is the same as the polycarbonate resin described above, and its preferred form is also the same thereas.

The content of the polycarbonate resin in the polycarbonate resin composition of the present embodiment is preferably 50% by mass or more and 100% by mass or less, more preferably 60% by mass or more and less than 100% by mass, further preferably 65% by mass or more and less than 100% by mass, based on the whole resin composition from the viewpoint of more effectively and reliably exerting the advantageous effects of the present invention.

The content of the antioxidant in the polycarbonate resin composition of the present embodiment is preferably 0.001% by mass or more and 1% by mass or less, more preferably 0.003% by mass or more and 1% by mass or less, further preferably 0.005% by mass or more and 1% by mass or less, based on the whole resin composition from the viewpoint of further suppressing degradation caused by heat or shear at the time of a molding process.

[Optical component]

The optical component of the present embodiment contains the polycarbonate resin described above or the polycarbonate resin composition described above.

The polycarbonate resin described above or the polycarbonate resin composition described above, contained in the optical component of the present embodiment has a small photoelastic coefficient, and a small in-plane phase difference associated with molecular orientation. The optical component of the present embodiment is therefore less likely to cause a stress double refraction and an orientation double refraction. Thus, the optical component of the present embodiment tends to be able to suppress a double refraction in use. Furthermore, the polycarbonate resin described above or the polycarbonate resin composition described above, contained in the optical component of the present embodiment has excellent heat resistance, as mentioned above. The optical component of the present embodiment is therefore less likely to be degraded over time and can be used for a long period as compared with conventional optical components.

Examples of the optical component according to the present embodiment include, but are not particularly limited to, optical lenses such as camera, telescope, microscope, projector, and car-mounted lenses, and optical films such as diffuser panels, light guide plates, polarizers, and phase difference films. The optical component of the present embodiment is obtained by appropriately subjecting the polycarbonate resin described above or the polycarbonate resin composition described above to processing such as molding so as to have a shape suitable for the purpose thereof.

[Method for producing polycarbonate resin]

Examples of the method for producing the polycarbonate resin of the present embodiment include, but are not particularly limited to, a method of copolymerizing epoxide and carbon dioxide, a method of ring-opening polymerizing a cyclic carbonate, and a method of condensation-polymerizing diol and carbon dioxide or carbonic acid ester. A method for producing the polycarbonate resin mentioned later is preferably used as a method for synthesizing the polycarbonate resin of the present embodiment from the viewpoint of more effectively and reliably exerting the advantageous effects of the present invention.

The method for producing the polycarbonate resin of the present embodiment preferably comprises the step of ring-opening polymerizing a cyclic carbonate mentioned later.

The cyclic carbonate is preferably a cyclic carbonate represented by the following formula (1L) (hereinafter, also referred to as a "cyclic carbonate (A1) ") :

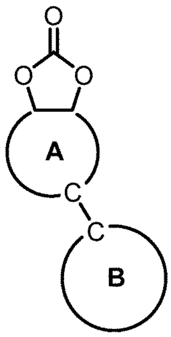
(1 L)

In the formula (1L), A and B are as described in the formula (1).

In the method for producing the polycarbonate resin of the present embodiment, the cyclic carbonate (A1) for use in ring-opening polymerization may be one cyclic carbonate (A1) used singly, or any two or more cyclic carbonates (A1) differing in A or B may be used in combination.

In the cyclic carbonate (A1) for use in ring-opening polymerization, a carbonate group preferably forms a 1,2-trans structure from the viewpoint of more effectively and reliably exerting the advantageous effects of the present invention.

In the method for producing the polycarbonate resin of the present embodiment, an additional cyclic carbonate may be used, in addition to the cyclic carbonate (A1).

Examples of the additional cyclic carbonate include a cyclic carbonate represented by the formula (3L) (hereinafter, also referred to as a "cyclic carbonate (A2) ") :

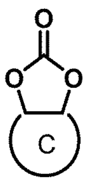
(3 L)

In the formula (3L), C is as described in the formula (2). In the method for producing the polycarbonate resin of the present embodiment, the cyclic carbonate (A2) for use in ring-opening polymerization may be one cyclic carbonate used singly, or any two or more cyclic carbonates differing in C may be used in combination.

(Polymerization initiator)

Examples of the polymerization initiator for ring-opening polymerizing the cyclic carbonates (A1) and (A2) include, but are not particularly limited to, acid catalysts, base catalysts, and enzyme catalysts. Examples of the base catalyst include, but are not particularly limited to, alkylmetals, metal alkoxides, metal amide, organic acid salts of metals, cyclic amine such as cyclic monoamine and cyclic diamine (particularly, cyclic diamine compounds having an amidine skeleton), triamine compounds having a guanidine skeleton, and heterocyclic compounds containing a nitrogen atom. Examples of the alkylmetal include, but are not particularly limited to, organolithium such as methyllithium, n-butyllithium, sec-butyllithium, tert-butyllithium, and phenyllithium, methyl magnesium halide, ethyl magnesium halide, propyl magnesium halide, phenyl magnesium halide, trimethyl aluminum, and triethyl aluminum. Among them, methyllithium, n-butyllithium, or sec-butyllithium is preferably used. Examples of the metal ion in the metal alkoxide include, but are not particularly limited to, alkali metal and alkaline earth metal ions. An alkali metal is preferred. Examples of the alkoxide ion include, but are not particularly limited to, methoxide, ethoxide, propoxide, butoxide, phenoxide, and benzyl oxide. The phenoxide or the benzyl oxide may have a substituent on the aromatic ring. Examples of the metal amide include, but are not particularly limited to, lithium amide, sodium amide, potassium amide, lithium bis(trimethylsilyl)amide, sodium bis(trimethylsilyl)amide, and potassium bis(trimethylsilyl)amide. Examples of the organic acid ion in the organic acid salt of a metal include, but are not particularly limited to, carboxylic acid ions having 1 to 10 carbon atoms. Examples of the metal in the organic acid salt of a metal include, but are not particularly limited to, lithium, sodium, potassium, rubidium, cesium, magnesium, calcium, and tin. Further examples of the base catalyst include, but are not particularly limited to, organic bases. Examples of the organic base include, but are not particularly limited to 1,4-diazabicyclo-[2.2.2]octane (DABCO), 1,8-diazabicyclo[5.4.0]undec-7-ene (DBU), 1,5-diazabicyclo[4.3.0]non-5-ene (DBN), 1,5,7-triazabicyclo [4.4.0]dec-5-ene (TBD), diphenylguanidine (DPG), N,N-dimethyl-4-aminopyridine (DMAP), imidazole, pyrimidine, purine, and phosphazene bases. The polymerization initiator of the present embodiment is preferably an alkylmetal, a metal alkoxide, or metal amide, more preferably a metal alkoxide or metal amide, from the viewpoint of more effectively and reliably exerting the advantageous effects of the present invention.

The amount of the polymerization initiator used in the polymerization step of the method for producing the polycarbonate resin of the present embodiment can be appropriately adjusted depending on the targeted molecular weight of the polycarbonate resin. The amount of the polymerization initiator used is preferably 0.0001 mol% or more and 4 mol% or less, more preferably 0.0001 mol% or more and 2 mol% or less, further preferably 0.0001 mol% or more and 1 mol% or less, in terms of the amount of a substance based on the ring-opening polymerizable monomer (the total amount of monomers such as the cyclic carbonate (A1) and the cyclic carbonate (A2)) from the viewpoint of controlling the weight-average molecular weight (Mw) of the polycarbonate resin within the range of 10,000 or larger and 500,000 or smaller. These polymerization initiators described above may be used singly, or in combinations of two or more thereof.

(Polymerization terminator)

In addition to the polymerization initiator, a polymerization terminator may be used from the viewpoint of controlling the average molecular weight of the resulting polycarbonate resin. Examples of the polymerization terminator include, but are not particularly limited to, inorganic acids and organic acids such as hydrochloric acid, sulfuric acid, nitric acid, methanesulfonic acid, benzenesulfonic acid, p-toluenesulfonic acid, trifluoromethanesulfonic acid, phosphoric acid, metaphosphoric acid, formic acid, acetic acid, propionic acid, butyric acid, lactic acid, citric acid, ascorbic acid, gluconic acid, oxalic acid, tartaric acid, Meldrum's acid, and benzoic acid.

(Additive)

In addition to the polymerization initiator, an additive may be used from the viewpoint of controlling the molecular weight of the resulting polymer, and from the viewpoint of exerting various characteristics by controlling a terminal structure. Examples of the additive include, but are not particularly limited to, monoalcohols such as methanol, ethanol, propanol, butanol, pentanol, hexanol, heptanol, nonanol, decanol, dodecanol, lauryl alcohol, myristyl alcohol, cetyl alcohol, stearyl alcohol, 5-norbornene-2-methanol, 1-adamantanol, 2-adamantanol, trimethylsilylmethanol, phenol, benzyl alcohol, and p-methylbenzyl alcohol, dialcohols such as ethylene glycol, 1,2-propanediol, 1,3-propanediol, 1,3-butanediol, 1,4-butanediol, hexanediol, nonanediol, tetramethylene glycol, and polyethylene glycol, polyhydric alcohols such as glycerol, sorbitol, xylitol, ribitol, erythritol, and triethanolamine as well as methyl lactate and ethyl lactate. These additives may be used singly, or in combinations of two or more thereof.

(Reaction temperature)

In the method for producing the polycarbonate resin of the present embodiment, the reaction temperature in the polymerization step is not particularly limited within a range that can produce the polycarbonate resin of the present embodiment, and is preferably -60°C or higher and 150°C or lower, more preferably -60°C or higher and 130°C or lower, further preferably -60°C or higher and 120°C or lower. When the reaction temperature in the polymerization step falls within the range described above, the weight-average molecular weight of the resulting polycarbonate resin is easier to control within the range of 10,000 or larger and 1,000,000 or smaller.

(Solvent)

The method for producing the polycarbonate resin of the present embodiment may or may not employ a solvent. Examples of the solvent include, but are not particularly limited to, ether solvents such as diethyl ether, diisopropyl ether,

dibutyl ether, diphenyl ether, tetrahydrofuran (THF), 2-methyltetrahydrofuran, 1,4-dioxane, cyclopentyl methyl ether, tert-butyl methyl ether, and propylene glycol monomethyl ether acetate, halogen solvents such as methylene chloride, chloroform, dichloromethane, dichloroethane, and trichloroethane, saturated hydrocarbon solvents such as hexane, heptane, octane, nonane, cyclohexane, and methylcyclohexane, aromatic hydrocarbon solvents such as toluene, xylene, o-xylene, m-xylene, p-xylene, and cresol, and ketone solvents such as acetone, 2-butanone, 2-pentanone, 3-pentanone, cyclopentanone, cyclohexanone, and methyl isobutyl ketone.

[Cyclic carbonate]

Since the polycarbonate resin is required to have various properties, a novel substance is demanded as the cyclic carbonate for use as a starting material for this resin. An object of the present embodiment is to provide a novel cyclic carbonate and a method for producing the same.

The cyclic carbonate of the present embodiment has a structure where an alicyclic skeleton is bonded to a 1,2-cycloalkylene carbonate skeleton via a single bond. The cyclic carbonate of the present embodiment having the configuration described above can be ring-opening polymerized to obtain polycarbonate excellent in heat resistance. The reason for this is probably, but is not limited to, as follows: most of cyclic carbonates having a conventional alicyclic structure are 1,2-cycloalkylene carbonate having no ring skeleton in a side chain and thus have insufficient heat resistance. By contrast, the cyclic carbonate of the present embodiment has a structure where an alicyclic skeleton is further bonded to a side chain of a 1,2-cycloalkylene carbonate skeleton. Therefore, the cyclic carbonate of the present embodiment presumably has excellent heat resistance while maintaining the ring-opening polymerizability of 1,2-cycloalkylene carbonate, with increase in the proportion of the ring skeleton in a molecule.

The 1,2-cycloalkylene carbonate skeleton means a skeleton with a cyclic carbonate group formed by a cycloalkane-1,2-diyl group and a carbonate group bonded to the 1,2-position.

Examples of the cycloalkane-1,2-diyl group include, but are not particularly limited to, a cyclopropane-1,2-diyl group, a cyclobutane-1,2-diyl group, a cyclopentane-1,2-diyl group, a cyclohexane-1,2-diyl group, a cycloheptane-1,2-diyl group, a cyclooctane-1,2-diyl group, a cyclononane-1,2-diyl group, and a cyclodecane-1,2-diyl group.

The alicyclic skeleton means a moiety having the structure of a cyclic aliphatic hydrocarbon. The alicyclic skeleton may be a monocyclic alicyclic skeleton or may be a polycyclic alicyclic skeleton and is preferably a polycyclic alicyclic skeleton. The alicyclic skeleton is preferably alicyclic skeleton B mentioned later.

The cyclic carbonate of the present embodiment is represented by the following formula (1L):

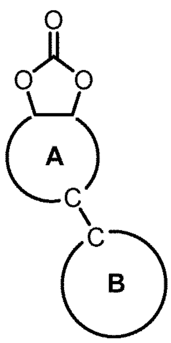

(1 L)

In the formula (1L), A and B are as described in the formula (1).

The structural unit represented by the formula (1) is preferably the following trans form.

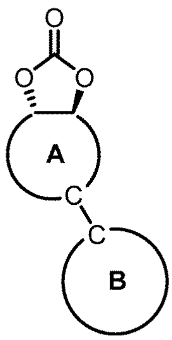

The above drawing illustrates a conformation, and if the formula involves a chiral center, any of R and S conformations may be used.

The cyclic carbonate of the present embodiment is preferably represented by the following formula (1LA):

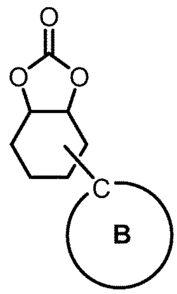

(1LA)

wherein B is an optionally substituted monovalent alicyclic moiety.

Preferred B in the formula (1LA) is as defined in the formula (1L). In the formula (1LA), when each group is substituted, examples of the substituent are also as defined in the formula (1L).

The cyclic carbonate of the present embodiment is preferably represented by the following formula (1LB) from the viewpoint of improving heat resistance and obtaining a low photoelastic coefficient:

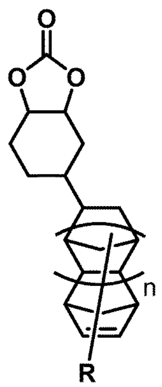

(1LB)

In the formula (1LB), R is a substituent, and n is an integer of 0 to 2. The substituent in the formula (1LB) is as defined in the formula (1L).

The cyclic carbonate of the present embodiment is preferably represented by the following formula (1LC) from the viewpoint of improving heat resistance and obtaining a low photoelastic coefficient:

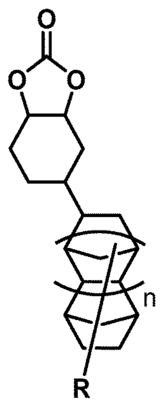

(1LC)

In the formula (1LC), R is a substituent, and n is an integer of 0 to 2. The substituent in the formula (1LC) is as defined in the formula (1L).

The cyclic carbonate having the structure of the formula (1L) is preferably a cyclic carbonate represented by

the following formula (1L-1):

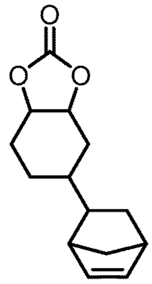

(1L-1)

the following formula (1L-2):

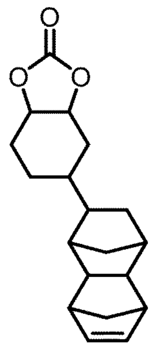

(1L-2)

the following formula (1L-3):

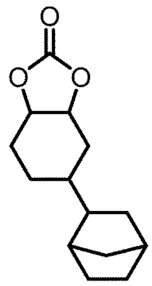

(1L-3)

or the following formula (1L-4):

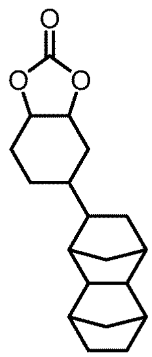

(1L-4)

from the viewpoint of more improving heat resistance and obtaining a lower photoelastic coefficient.

The properties of the cyclic carbonate represented by the formula (1L-1) or the formula (1L-2) mentioned above can be tuned by the functional group conversion of an olefin moiety.

The cyclic carbonate composition of the present embodiment composition preferably contains a cyclic carbonate

represented by the formula (1L-1) and a cyclic carbonate represented by the formula (1L-2). The cyclic carbonate composition of the present embodiment containing the cyclic carbonate represented by the formula (1L-1) and the cyclic carbonate represented by the formula (1L-2) permits introduction of various substituents by the functional group conversion of an olefin moiety and facilitates tuning properties such as heat resistance and a photoelastic coefficient.

The molar ratio between the cyclic carbonate represented by the formula (1L-1) and the cyclic carbonate represented by the formula (1L-2) (cyclic carbonate represented by the formula (1L-1)/cyclic carbonate represented by the formula (1L-2)) in the cyclic carbonate composition of the present embodiment is not particularly limited and is preferably, for example, 10/90 to 90/10, 20/80 to 80/20, 30/70 to 70/30, or 40/60 to 60/40.

The cyclic carbonate represented by the formula (1L) is preferably a cyclic carbonate represented by the formula (1L-3) or the formula (1L-4) mentioned above from the viewpoint of more improving heat resistance.

The cyclic carbonate of composition the present embodiment preferably contains a cyclic carbonate represented by the formula (1L-3) and a cyclic carbonate represented by the formula (1L-4) from the viewpoint of more improving heat resistance.

The molar ratio between the cyclic carbonate represented by the formula (1L-3) and the cyclic carbonate represented by the formula (1L-4) (cyclic carbonate represented by the formula (1L-3)/cyclic carbonate represented by the formula (1L-4)) in the cyclic carbonate composition of the present embodiment is not particularly limited and is preferably, for example, 10/90 to 90/10, 20/80 to 80/20, 30/70 to 70/30, or 40/60 to 60/40.

More specific examples of the cyclic carbonate having the structure of the formula (1L) include 4-(5-norbornen-2-yl)-trans-1,2-cyclohexene carbonate, 4-(5-norbornan-2-yl)-trans-1,2-cyclohexene carbonate, 4-(5-tetracyclododecen-2-yl)-trans-1,2-cyclohexene carbonate, 4-(5-tetracyclododecan-2-yl)-trans-1,2-cyclohexene carbonate, and 4-(1,4,5,6,7-pentamethyl-5-norbornen-2-yl)-trans-1,2-cyclohexene carbonate. Among them, more specifically, 4-(5-norbornen-2-yl)-trans-1,2-cyclohexene carbonate, 4-(5-norbornan-2-yl)-trans-1,2-cyclohexene carbonate, 4-(5-tetracyclododecen-2-yl)-trans-1,2-cyclohexene carbonate, and 4-(5-tetracyclododecan-2-yl)-trans-1,2-cyclohexene carbonate are more preferred from the viewpoint of more improving heat resistance and obtaining a lower photoelastic coefficient.

[Method for producing cyclic carbonate]

The method for producing the cyclic carbonate of the present embodiment preferably comprises a step of reacting a cyclic carbonate represented by the following formula (2L) with a compound having a conjugated diene moiety:

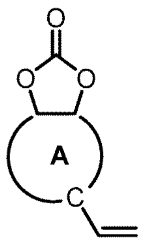
(2 L)

wherein A is an optionally substituted trivalent alicyclic moiety.

Preferred A in the formula (2L) is as defined in the formula (1L). Examples of the substituent in the formula (2L) are also as defined in the formula (1L).

Examples of the compound having a conjugated diene moiety for use in the production method include, but are not particularly limited to, 1,3-butadiene, 2,3-dimethyl-1,3-butadiene, 1-methoxy-3-trimethylsilyloxy-1,3-butadiene, trans-3-(tert-butyldimethylsilyloxy)-N,N-dimethyl-1,3-butadiene-1-amine, 1,3-pentadiene, 2,4-hexadiene, 3-methyl-1,3-pentadiene, 1,3-cyclopentadiene, 1,3-cyclohexadiene, 2-trimethylsilyloxy-1,3-cyclohexadiene, 1,3,5,5-tetramethyl-1,3-cyclohexadiene, α-terpinene, α-phellandrene, 1,2,3,4,5-pentamethylcyclopentadiene, 5-trimethylsilyl-1,2,3,4,5-pentamethyl-1,3-cyclopentadiene, 1,2,3,4-tetraphenyl-1,3-cyclopentadiene, 1,2,3,4,5-pentaphenyl-1,3-cyclopentadiene, and 1,3-cyclooctadiene.

The compound having a conjugated diene moiety according to the present embodiment may be formed during reaction by thermal decomposition or the like in the process of producing the cyclic carbonate. For example, instead of 1,3-cyclopentadiene, dicyclopentadiene is thermally decomposed during reaction to form 1,3-cyclopentadiene, which may be used.

In the method for producing the cyclic carbonate according to the present embodiment, the cyclic carbonate according to the present embodiment can be purified by purification operations such as distillation, column chromatography, and recrystallization from a reaction product obtained in the reaction step.

[Purpose of cyclic carbonate]

The cyclic carbonate according to the present embodiment can be used as a starting material for the polycarbonate resin. The polycarbonate resin is obtained by ring-opening polymerizing the cyclic carbonate using an initiator such as anionic polymerization initiator. For other purposes, the cyclic carbonate can be used as an electrolyte in a lithium ion secondary battery or the like, or an additive for an electrolyte.

Examples

The present invention will be described further specifically with reference to Examples and Comparative Examples. However, the present invention is not limited by these Examples, etc. by any means.
Various abbreviations are as follows.

T6C: trans-1,2-cyclohexene carbonate
VCHC: 4-vinyl-trans-1,2-cyclohexene carbonate
NORT: 4-(5-norbornen-2-yl)-trans-1,2-cyclohexene carbonate
NORT-H: 4-(5-norbornan-2-yl)-trans-1,2-cyclohexene carbonate
TCDT: 4-(5-tetracyclododecen-2-yl)-trans-1,2-cyclohexene carbonate
TCDT-H: 4-(5-tetracyclododecan-2-yl)-trans-1,2-cyclohexene carbonate
$Me_5$NORT: 4-(1,4,5,6,7-pentamethyl-5-norbornen-2-yl)-trans-1,2-cyclohexene carbonate

In the present specification, the properties of a cyclic carbonate were measured as follows.

($^1$H-NMR measurement)

The $^1$H-NMR spectrum of a cyclic carbonate was obtained by NMR measurement using an NMR apparatus manufactured by JEOL Ltd. (product name: ECZ400S), and a TFH probe. The reference peak of a deuterated solvent was $\delta_H$ = 7.26 ppm in the case of using chloroform-d. The measurement was performed with the number of scans set to 32.

(High-resolution mass spectrometry: HR-MS)

A cyclic carbonate was subjected to high-resolution mass spectrometry using a GC/Q-TOFMS apparatus manufactured by Agilent Technologies, Inc. (product name: Agilent 7890GC/7200 Accurate-MassQ-TOF GC/MS).
More specifically, an acetone solution of the cyclic carbonate was prepared so as to have a sample concentration of 500 ppm (2500 ppm in Example L1). Agilent J&W GC column manufactured by Agilent Technologies, Inc. (product name: DB-1) was used, and the column temperature was kept at 40°C for 5 minutes, then elevated to 300°C at 20°C/min, and kept at 300°C for 12 minutes. In this operation, 20% methane was used as a reactive gas, and a proton adduct of each cyclic carbonate detected by chemical ionization was subjected to high-resolution mass spectrometry. A calculated value was described as "calc.", and a measured value was described as "found".
(Measurement of weight-average molecular weight (Mw) and number-average molecular weight (Mw))
A solution of 2.0 g of tetrahydrofuran added to 0.02 g of a polycarbonate resin was used as a measurement sample. The weight-average molecular weight of the polycarbonate resin was measured using a high-performance GPC apparatus (manufactured by Tosoh Corp., product name "HLC-8420GPC"). The columns used were TSK guard columns SuperH-H, TSKgel SuperHM-H, TSKgel SuperHM-H, TSKgel SuperH2000, and TSKgel SuperH1000 manufactured by Tosoh Corp. (all of which are product names of Tosoh Corp.) connected in series. The column temperature was 40°C, and tetrahydrofuran was used as a mobile phase. Analysis was conducted at a rate of 0.60 mL/min. The detector used was an RI detector. A calibration curve was prepared using polystyrene standard samples manufactured by Polymer Standards Service GmbH (molecular weight: 2520000, 1240000, 552000, 277000, 130000, 66000, 34800, 19700, 8680, 3470, 1306, and 370) as standard samples. The number-average molecular weight and the weight-average molecular weight of the polycarbonate resin were determined on the basis of the calibration curve thus prepared.

(Measurement of glass transition temperature (Tg))

Approximately 5 mg of a polycarbonate resin obtained in each of Examples and Comparative Examples mentioned later was used as a measurement sample. Its glass transition temperature was measured using a differential scanning calorimetric apparatus manufactured by PerkinElmer Co., Ltd. (product name "DSC8500") under conditions involving a nitrogen gas flow rate of 20 mL/min.
More specifically, the sample was kept at 40°C for 3 minutes and then warmed from 40°C to 210°C at 20°C/min for

primary temperature elevation to completely melt the sample. Then, the sample was cooled from 210°C to 40°C at 50°C/min and kept at 40°C for 5 minutes. Subsequently, the sample was warmed from 40°C to 200°C at 10°C/min for secondary temperature elevation. The point of intersection (midpoint glass transition temperature) between a partial curve of step-like change in a DSC curve stretched by the secondary temperature elevation and a straight line pitched at equal distances in the ordinate direction from extended lines of respective tangents was regarded as a glass transition temperature (Tg).

(Measurement of in-plane phase difference, rate of orientation double refraction, and temperature dependence of orientation double refraction)

<<Preparation of unstretched sample and stretched sample>>

First, an unstretched sample of a polycarbonate resin was prepared using a vacuum compression molding machine. Specifically, the sample was prepared as described below. The polycarbonate resin was placed in a 25 to 150 μm thick polyimide frame and sandwiched between two polyimide films, two aluminum sheets and two iron sheets to obtain a laminate. In this respect, the order of lamination for the laminate was the iron sheet, the aluminum sheet, the polyimide film, the polyimide frame, the polyimide film, the aluminum sheet, and the iron sheet. The laminate described above was loaded in a vacuum compression molding machine (manufactured by Shinto Metal Industries, Ltd., model SFV-30), preheated at a predetermined temperature under reduced pressure (10 kPa) for 5 minutes, and then compressed at the predetermined temperature for 10 minutes under conditions involving a press pressure of 10 MPa while the reduced pressure conditions were maintained. After cancelation of the reduced pressure and the press pressure, the compressed laminate was transferred to a compression molding machine for cooling (manufactured by Shinto Metal Industries, Ltd., model AYS-10) and solidified by cooling to obtain a press film having a thickness of 20 to 250 μm. The obtained press film was cured for 24 hours in a constant temperature and humidity room of 23°C and 50% humidity to obtain an unstretched sample.

Next, the unstretched sample was cut into a width of 30 mm and a length of 50 mm and placed in a tensile test jig. The tensile test jig was loaded in a tester manufactured by Shimadzu Corp. (product name "Autograph AG-5kNXPlus") connected with a thermostat bath (product name "TCR1W-200T"), and uniaxially freely stretched under the following conditions: the stretching conditions involved a distance of 30 mm between chucks, a stretching temperature of Tg + 20°C, a stretching rate of 50 mm/min, and a stretch ratio of 100% (which means two-fold stretching). The sample thus uniaxially freely stretched was immediately taken out thereof and cooled at room temperature and then cured for 24 hours in a constant temperature and humidity room of 23°C and 50% humidity to obtain a stretched film having a thickness of 10 to 500 μm. A few samples of the stretched film obtained by the procedures were prepared. Then, the thickness of each film was measured using a thickness gauge (manufactured by Mitutoyo Corp., "Digimatic Thickness Gauge").

<<Measurement conditions for in-plane phase difference and rate of orientation double refraction>>

An in-plane phase difference and a rate of orientation double refraction were measured as described below using the 100% uniaxially stretched film. First, the absolute value of the in-plane phase difference of each stretched film was measured at a wavelength of 587 nm using a phase difference measurement apparatus manufactured by Oji Scientific Instruments Co., Ltd. (product name "KOBRA-WR"). Then, the rate of orientation double refraction was calculated using the obtained absolute value of the in-plane phase difference and the thickness of each stretched film. The absolute value of the in-plane phase difference per 100 μm in thickness was calculated from the thickness of the stretched film on the basis of the obtained rate of orientation double refraction, and used as the absolute value of the in-plane phase difference in terms of a thickness of 100 μm. The rate of orientation double refraction, the absolute value of the in-plane phase difference, the absolute value of the in-plane phase difference in terms of a thickness of 100 μm, and the thickness of the stretched film satisfy the following expressions (A), (B) and (C):

$$\Delta n = nx - ny \qquad (A)$$

$$Re = \Delta n \times d \qquad (B)$$

$$Re_{100}\ (nm) = \Delta n \times 1.0 \times 10^5 \qquad (C)$$

($\Delta n$: rate of orientation double refraction, nx: refractive index in the direction of stretching, ny: refractive index in a direction perpendicular to the direction of stretching within the plane of the sample, Re: absolute value of the in-plane phase difference, $Re_{100}$: absolute value of the in-plane phase difference in terms of a thickness of 100 μm, d: thickness of the stretched film)

<<Measurement of temperature dependence of orientation double refraction>>

The temperature dependence of orientation double refraction was measured using the uniaxially stretched film obtained as described above. First, the prepared film was loaded in a temperature controller and left at a predetermined temperature for 15 minutes for stabilization, followed by repeated measurement of double refractions from 30°C to 60°C at 10°C intervals. The amount of change in double refraction at each temperature corresponds to the temperature dependence of orientation double refraction. Therefore, the temperature dependence of orientation double refraction, $d\Delta n/dT$, was calculated by determining the slope of an approximate straight line by the least squares method.

(Measurement of photoelastic coefficient)

The unstretched sample obtained in the section "Preparation of unstretched sample and stretched sample" was cut into a width of 6 mm and a length of 30 mm and used as a sample. Its photoelastic coefficient was measured as described below. For the detailed measurement method, see Polymer Engineering and Science 1999, 39, 2349-2357. Specifically, the sample described above was arranged in a film tensile apparatus (manufactured by Imoto Machinery Co., Ltd.) placed in a constant temperature and humidity room of 23°C and 50% humidity such that the distance between chucks was 20 mm. Subsequently, a double refraction measurement apparatus (manufactured by Otsuka Electronics Co., Ltd., product name "RETS-100") was arranged such that the light path of the double refraction measurement apparatus was positioned at the central portion of the sample. The distance between chucks was 20 mm, and the chuck movement speed was 0.1 mm/min. The rate of double refraction of the test piece was measured at a wavelength of 550 nm while elongational stress was applied thereto. The photoelastic coefficient ($Pa^{-1}$) was calculated from the relationship between the measured rate of double refraction and the elongational stress by use of the least-square method. For the calculation, data on elongational stress $\sigma$ of $2.5\ \text{MPa} \leq \sigma \leq 10\ \text{MPa}$ was used. The rate of double refraction, the elongational stress, and the photoelastic coefficient satisfy the following expressions (D) and (E):

$$\Delta n = nx - ny \quad (D)$$

$$C = \Delta n / \sigma \quad (E)$$

($\Delta n$: rate of double refraction, nx: refractive index in the direction of stretching, ny: refractive index in a direction perpendicular to the direction of stretching within the plane of the sample, C: photoelastic coefficient, $\sigma$: elongational stress)

[Synthesis Example 1: VCHC]

4-Vinylcyclohexene oxide (485 g, 3.91 mol) and ion-exchange water (4.85 L) were added to a 20 L five-neck flask in an argon atmosphere and stirred at room temperature using a mechanical stirrer. The flask was heated at an internal temperature of 95°C for 16 hours using a mantle heater. After the flask was allowed to cool, the reaction solution was concentrated under reduced pressure using an evaporator, and toluene (4 L) was added to the concentrate, followed by azeotropy. This azeotropy operation was carried out a total of six times to obtain 4-vinyl-trans-1,2-cyclohexanediol (hereinafter, also referred to as VCHDL; 551 g).

Subsequently, VCHDL (551 g, 3.87 mol) and tetrahydrofuran (5.52 L) were added to a five-neck flask in an argon atmosphere and stirred at room temperature using a mechanical stirrer. The flask was cooled by dipping in a salt ice bath, and ethyl chloroformate (842 g, 7.76 mol) and triethylamine (1060 g, 10.48 mol) were added dropwise to the flask. The flask was taken out of the ice bath and left such that the temperature was spontaneously elevated to room temperature. After stirring at room temperature for 19 hours, the flask was cooled again by dipping in a salt ice bath, and ethyl chloroformate (210 g, 1.94 mol) and triethylamine (265 g, 2.62 mol) were added dropwise to the flask. The flask was taken out of the ice bath and left such that the temperature was spontaneously elevated to room temperature. After stirring at room temperature for 20 hours, the reaction solution was filtered under reduced pressure, and the filter cake was rinsed with tetrahydrofuran/toluene = 2/1 (3 L × 3 times). The filtrate was concentrated under reduced pressure using an evaporator, and the obtained concentrate was dissolved in chloroform (4.5 L). Ion-exchange water (4.5 L) was added to the solution, and the mixture was stirred and separated into organic and aqueous layers to recover an organic layer. The recovered organic layer was washed again with ion-exchange water (4.5 L). The recovered organic layer was dried over magnesium sulfate and filtered. The organic layer was concentrated under reduced pressure using an evaporator, and the obtained concentrate was purified by silica gel column chromatography to obtain VCHC (569 g).

[Synthesis Example 2: TCDT]

VCHC (501 g, 3 mol) and dicyclopentadiene (2484 g, 18 mol) were added to a 3 L autoclave. The autoclave was heated to an internal temperature of 200°C, and reaction was continued for 3 days. After the autoclave was allowed to cool to an internal temperature of 60°C, the contents were recovered, added into chloroform (5.5 L), and dissolved therein. This solution was slowly poured into methanol (20 L) and stirred for 1 hour. Deposits were filtered off under reduced pressure and rinsed with methanol (1.5 L). The filtrate was concentrated under reduced pressure, and the concentrate was then separated into two fractions, A and B, by silica gel column chromatography to obtain 490 g and 231 g of concentrates from the fractions A and B, respectively. The concentrate (490 g) of the fraction A was subjected again to silica gel chromatography to concentrate a fraction containing the compound of interest. Since a white solid started to be deposited during concentration, the concentration was discontinued. The solid was recovered by filtration under reduced pressure and dried under reduced pressure at 50°C to obtain TCDT (25 g).

[Synthesis Example 3: NORT]

The filtrate after removal of the white solid in Example 1 was concentrated under reduced pressure, and the obtained concentrate (144 g) was combined with the concentrate (231 g) of the fraction B obtained in Example 1. The mixture was distilled under reduced pressure at 220°C/0.6 to 2 hPa to obtain 168 g of a distillate. The obtained distillate was purified by silica gel column chromatography and dried under pressure at 50°C to obtain NORT (102 g).

[Synthesis Example 4: NORT-H]

NORT (4.78 g, 20.4 mmol) and chloro(1,5-cyclooctadiene)iridium(I) dimer (0.145 g, 0.216 mmol) were weighed into a 50 mL three-neck flask, and the flask was purged with nitrogen. Toluene (deoxidized grade manufactured by FUJIFILM Wako Pure Chemical Corp.; 10 mL) and 2-propanol (deoxidized grade manufactured by FUJIFILM Wako Pure Chemical Corp.; 10 mL) were weighed and added to the flask. Further, 1,2-bis(dicyclohexylphosphino)ethane (0.187 g, 0.442 mmol) was weighed and added to the flask. The flask was dipped in an oil bath of 100°C and heated for 6 hours with stirring using a magnetic stirrer. After the flask was allowed to cool, the contents were transferred to a 50 mL flask, and the solvent was distilled off using an evaporator. The concentrated residue was purified by silica gel column chromatography, and a fraction containing the compound of interest was recovered to obtain 5.01 g of a concentrate. This concentrate was dissolved in an ethyl acetate (2 mL)/heptane (10 mL) mixed solvent, and the solution was transferred to a freezer of -30°C and left standing overnight. A deposited solid was recovered by filtration under reduced pressure, then washed with heptane, and dried under reduced pressure at 60°C to obtain NORT-H (3.45 g).

[Synthesis Example 5: TCDT-H]

TCDT-H (2.27 g) was obtained through the same reaction as in Synthesis Example 3 except that TCDT (3.02 g) was used instead of NORT.

[Example 1]

T6C (1.05 g, 7.36 mmol) and a mixture of NORT:TCDT = 2:3 (2.01 g, 7.35 mmol) were weighed into a 50 mL three-neck flask, and the flask was purged with nitrogen. Dehydrated m-xylene (11.9 g) was added into the flask, and the mixture was then stirred using a magnetic stirrer for complete dissolution. The flask was dipped in a thermostat bath of 25°C. A tetrahydrofuran solution of potassium tert-butoxide (1.0 M, 28 μL, 0.28 mmol) was added thereto, and the mixture was stirred at 25°C for 1 hour. The reaction was terminated by the addition of acetic acid (0.010 g) (polymerization solution).

Subsequently, a reprecipitation operation was performed as described below for the evaluation of methanol-insoluble matter. The polymerization solution was diluted by the addition of acetone (61 g) and chloroform (31 g). The diluted solution was added into 400 g of methanol to deposit a polycarbonate resin. The deposited polycarbonate resin was recovered by filtration under reduced pressure and washed with methanol. The obtained polycarbonate resin was dried in vacuum at 100°C for 2 hours to obtain a polycarbonate resin (2.54 g). The proportions of T6C, NORT, and TCDT in the polycarbonate resin were calculated as 52 mol%, 20 mol%, and 28 mol%, respectively, from the $^1$H-NMR spectrum of the obtained polycarbonate resin. The $^1$H-NMR spectrum is shown in Figure 1.

[Example 2]

T6C (0.75 g, 5.28 mmol), a mixture of NORT-H:TCDT-H = 2:3 (1.46 g, 5.29 mmol), and dehydrated m-xylene (8.71 g) were weighed into a 25 mL three-neck flask and dehydrated using molecular sieves 4A (monomer solution).

Another 25 mL three-neck flask was purged with nitrogen, and the monomer solution (10.04 g) was then collected into this flask. The flask was dipped in a thermostat bath of 25°C. A tetrahydrofuran solution of potassium tert-butoxide (1.0 M, 20 μL, 0.020 mmol) was added thereto, and the mixture was stirred at 25°C for 30 minutes. The reaction was terminated by the addition of acetic acid (0.011 g) (polymerization solution).

Subsequently, for the evaluation of methanol-insoluble matter, the polymerization solution was diluted by the addition of chloroform (39 g). The diluted solution was added into 284 g of methanol to deposit a polycarbonate resin. The deposited polycarbonate resin was recovered by filtration under reduced pressure and washed with methanol. The obtained polycarbonate resin was dried in vacuum at 100°C for 2 hours to obtain a polycarbonate resin (1.87 g). The proportions of T6C, NORT-H, and TCDT-H in the polycarbonate resin were calculated as 52 mol%, 26 mol%, and 22 mol%, respectively, from the $^{1}$H-NMR spectrum of the obtained polycarbonate resin. The $^{1}$H-NMR spectrum is shown in Figure 2.

[Example 3]

NORT-H (3.01 g, 12.7 mmol) and dehydrated m-xylene (12.2 g) were weighed into a 50 mL three-neck flask and dehydrated using molecular sieves 4A (monomer solution). Another 50 mL three-neck flask was purged with nitrogen, and the monomer solution (11.96 g) was then collected into this flask. The flask was dipped in a thermostat bath of 25°C. A tetrahydrofuran solution of potassium tert-butoxide (1.0 M, 33 μL, 0.033 mmol) was added thereto, and the mixture was stirred at 25°C for 15 minutes. The reaction was terminated by the addition of acetic acid (0.010 g) (polymerization solution).

Subsequently, for the evaluation of methanol-insoluble matter, the polymerization solution was diluted by the addition of m-xylene (46 g). The diluted solution was added into 482 g of methanol to deposit a polycarbonate resin. The deposited polycarbonate resin was recovered by filtration under reduced pressure and washed with methanol. The obtained polycarbonate resin was dried in vacuum at 80°C for 4 hours to obtain a polycarbonate resin (2.21 g). The $^{1}$H-NMR spectrum of the obtained polycarbonate resin is shown in Figure 3.

[Example 4]

T6C (0.32 g, 2.24 mmol), NORT-H (2.11 g, 8.93 mmol), and dehydrated m-xylene (9.81 g) were weighed into a 25 mL three-neck flask and dehydrated using molecular sieves 4A (monomer solution). Another 50 mL three-neck flask was purged with nitrogen, and the monomer solution (8.13 g) was then collected into this flask (the solution contained T6C: 0.21 g, NORT-H: 1.40 g, ad m-xylene: 6.52 g). The flask was dipped in a thermostat bath of 25°C. A tetrahydrofuran solution of potassium tert-butoxide (1.0 M, 4 μL, 0.004 mmol) was added thereto, and the mixture was stirred at 25°C for 30 minutes. The reaction was terminated by the addition of acetic acid (0.0040 g) (polymerization solution).

Subsequently, for the evaluation of methanol-insoluble matter, the polymerization solution was diluted by the addition of chloroform (22 g). The diluted solution was added into 287 g of methanol to deposit a polycarbonate resin. The deposited polycarbonate resin was recovered by filtration under reduced pressure and washed with methanol. The obtained polycarbonate resin was dried in vacuum at 100°C for 6 hours to obtain a polycarbonate resin (1.46 g). The proportions of T6C and NORT-H in the polycarbonate resin were calculated as 19 mol% and 81 mol%, respectively, from the $^{1}$H-NMR spectrum of the obtained polycarbonate resin. The $^{1}$H-NMR spectrum is shown in Figure 4.

[Example 5]

T6C (0.77 g, 5.40 mmol), TCDT-H (0.73 g, 2.42 mmol), and dehydrated m-xylene (12.50 g) were weighed into a 25 mL three-neck flask and dehydrated using molecular sieves 4A (monomer solution). Another 50 mL three-neck flask was purged with nitrogen, and the monomer solution (10.52 g) was then collected into this flask (the solution contained T6C: 0.58 g, TCDT-H: 0.55 g, and m-xylene: 9.39 g). The flask was dipped in a thermostat bath of 25°C. A tetrahydrofuran solution of potassium tert-butoxide (1.0 M, 5 μL, 0.005 mmol) was added thereto, and the mixture was stirred at 25°C for 30 minutes. The reaction was terminated by the addition of acetic acid (0.0022 g) (polymerization solution).

Subsequently, for the evaluation of methanol-insoluble matter, the polymerization solution was diluted by the addition of m-xylene (13 g). The diluted solution was added into 344 g of methanol to deposit a polycarbonate resin. The deposited polycarbonate resin was recovered by filtration under reduced pressure and washed with methanol. The obtained polycarbonate resin was dried in vacuum at 100°C for 6 hours to obtain a polycarbonate resin (0.97 g). The proportions of T6C and TCDT-H in the polycarbonate resin were calculated as 59 mol% and 41 mol%, respectively, from the $^{1}$H-NMR spectrum of the obtained polycarbonate resin. The $^{1}$H-NMR spectrum is shown in Figure 5.

[Example 6]

T6C (3.69 g, 25.9 mmol), NORT-H (2.03 g, 8.59 mmol), TCDT-H (1.59 g, 5.26 mmol), and dehydrated m-xylene

(29.30 g) were weighed into a 50 mL three-neck flask and dehydrated using molecular sieves 4A (monomer solution). Another 50 mL three-neck flask was purged with nitrogen, and the monomer solution (31.74 g) was then collected into this flask (the solution contained T6C: 3.19 g, NORT-H: 1.76 g, TCDT-H: 1.38 g, and m-xylene: 25.4 g). The flask was dipped in a thermostat bath of 25°C. A tetrahydrofuran solution of potassium tert-butoxide (1.0 M, 17 μL, 0.017 mmol) was added thereto, and the mixture was stirred at 25°C for 1 hour. The reaction was terminated by the addition of acetic acid (0.0061 g) (polymerization solution).

Subsequently, for the evaluation of methanol-insoluble matter, the polymerization solution was diluted by the addition of chloroform (119.56 g). The diluted solution was added into 730 g of methanol to deposit a polycarbonate resin. The deposited polycarbonate resin was recovered by filtration under reduced pressure and washed with methanol. The obtained polycarbonate resin was dried in vacuum at 100°C for 4 hours to obtain a polycarbonate resin (5.42 g). The proportions of T6C, NORT-H, and TCDT-H in the polycarbonate resin were calculated as 67 mol%, 21 mol%, and 12 mol%, respectively, from the $^1$H-NMR spectrum of the obtained polycarbonate resin. The $^1$H-NMR spectrum is shown in Figure 6.

[Example 7]

T6C (4.81 g, 33.8 mmol), NORT-H (1.01 g, 4.27 mmol), TCDT-H (1.44 g, 4.76 mmol), and dehydrated m-xylene (29.04 g) were weighed into a 50 mL three-neck flask and dehydrated using molecular sieves 4A (monomer solution). Another 50 mL three-neck flask was purged with nitrogen, and the monomer solution (32.06 g) was then collected into this flask (the solution contained T6C: 4.25 g, NORT-H: 0.89 g, TCDT-H: 1.28 g, and m-xylene: 25.6 g). The flask was dipped in a thermostat bath of 25°C. A tetrahydrofuran solution of potassium tert-butoxide (1.0 M, 19 μL, 0.019 mmol) was added thereto, and the mixture was stirred at 25°C for 1 hour. The reaction was terminated by the addition of acetic acid (0.0062 g) (polymerization solution).

Subsequently, for the evaluation of methanol-insoluble matter, the polymerization solution was diluted by the addition of chloroform (116.26 g). The diluted solution was added into 718 g of methanol to deposit a polycarbonate resin. The deposited polycarbonate resin was recovered by filtration under reduced pressure and washed with methanol. The obtained polycarbonate resin was dried in vacuum at 100°C for 4 hours to obtain a polycarbonate resin (5.33 g). The proportions of T6C, NORT-H, and TCDT-H in the polycarbonate resin were calculated as 77 mol%, 11 mol%, and 12 mol%, respectively, from the $^1$H-NMR spectrum of the obtained polycarbonate resin. The $^1$H-NMR spectrum is shown in Figure 7.

[Example 8]

T6C (3.99 g, 28.1 mmol), TCDT-H (2.72 g, 9.00 mmol), and dehydrated m-xylene (26.93 g) were weighed into a 50 mL three-neck flask and dehydrated using molecular sieves 4A (monomer solution). Another 50 mL three-neck flask was purged with nitrogen, and the monomer solution (29.79 g) was then collected into this flask (the solution contained T6C: 3.54 g, TCDT-H: 2.41 g, and m-xylene: 23.8 g). The flask was dipped in a thermostat bath of 25°C. A tetrahydrofuran solution of potassium tert-butoxide (1.0 M, 16 μL, 0.016 mmol) was added thereto, and the mixture was stirred at 25°C for 1 hour. The reaction was terminated by the addition of acetic acid (0.0053 g) (polymerization solution).

Subsequently, for the evaluation of methanol-insoluble matter, the polymerization solution was diluted by the addition of chloroform (110 g). The diluted solution was added into 799 g of methanol to deposit a polycarbonate resin. The deposited polycarbonate resin was recovered by filtration under reduced pressure and washed with methanol. The obtained polycarbonate resin was dried in vacuum at 100°C for 4 hours to obtain a polycarbonate resin (5.30 g). The proportions of T6C and TCDT-H in the polycarbonate resin were calculated as 73 mol% and 27 mol%, respectively, from the $^1$H-NMR spectrum of the obtained polycarbonate resin. The $^1$H-NMR spectrum is shown in Figure 8.

[Comparative Example 1]

A polycarbonate resin was obtained by polymerization reaction and a reprecipitation operation by the same methods as in Example 3 except that T6C was used instead of NORT-H.

Table 1 shows the properties of the polycarbonate resins obtained in Examples and Comparative Example.

[Table 1]

| | Example 1 | Example 2 | Example 3 | Example 4 | Example 5 | Example 6 | Example 7 | Example 8 | Comparative Example 1 |
|---|---|---|---|---|---|---|---|---|---|
| Monomer A | T6C | T6C | - | T6C | T6C | T6C | T6C | T6C | T6C |
| Monomer B | NORT | NORT-H | NORT-H | NORT-H | - | NORT-H | NORT-H | - | - |
| Monomer C | TCDT | TCDT-H | - | - | TCDT-H | TCDT-H | TCDT-H | TCDT-H | - |
| Composition of A in polymer, mol% | 52 | 52 | - | 19 | 59 | 67 | 77 | 73 | 100 |
| Composition of B in polymer, mol% | 20 | 26 | 100 | 81 | - | 21 | 11 | - | - |
| Composition of C in polymer, mol% | 28 | 22 | - | - | 41 | 12 | 12 | 27 | - |
| Mw | 69000 | 74000 | 415000 | 464000 | 219000 | 311000 | 287000 | 243000 | 201000 |
| Mn | 41000 | 42000 | 151000 | 161000 | 72000 | 101000 | 102000 | 80000 | 94000 |
| Mw/Mn | 1.7 | 1.8 | 2.7 | 2.9 | 3.1 | 3.1 | 2.8 | 3.1 | 2.1 |
| Glass transition temperature, °C | 169 | 170 | 176 | 166 | 164 | 150 | 144 | 149 | 120 |
| Photoelastic coefficient, $\times 10^{-12}$/Pa | 0.6 | 0.7 | - | 0.9 | 0.1 | 1.1 | 1.1 | 0.9 | 2.0 |
| In-plane phase difference, nm/100 μm | 63 | 36 | - | 73 | 12 | 84 | 44 | 34 | 45 |
| Temperature dependence of orientation double refraction (dΔn/dT) | - | $0.042\times 10^{-5}$ | - | $-0.012\times 10^{-5}$ | $0.032\times 10^{-5}$ | - | - | - | $-0.014\times 10^{-5}$ |

From Table 1, the polycarbonate resins of Examples had a much lower photoelastic coefficient and a much higher glass transition temperature than those of the polycarbonate resin of Comparative Example and were thus found to be excellent in optical characteristics and heat resistance.

[Example L1: TCDT]

VCHC (501 g, 3 mol) and dicyclopentadiene (hereinafter, also referred to as DCPD; 2484 g, 18 mol) were added to a 3 L autoclave. The autoclave was heated to an internal temperature of 200°C, and reaction was continued for 3 days. After the autoclave was allowed to cool to an internal temperature of 60°C, the contents were recovered, added into chloroform (5.5 L), and dissolved therein. This solution was slowly poured into methanol (20 L) and stirred for 1 hour. Deposits were filtered off under reduced pressure and rinsed with methanol (1.5 L). The filtrate was concentrated under reduced pressure, and the concentrate was then separated into two fractions, A and B, by silica gel column chromatography to obtain 490 g and 231 g of concentrates from the fractions A and B, respectively.

The concentrate (490 g) of the fraction A was subjected again to silica gel chromatography to concentrate a fraction containing the compound of interest. Since a white solid started to be deposited during concentration, the concentration was discontinued. The solid was recovered by filtration under reduced pressure and dried under reduced pressure at 50°C to obtain TCDT (25 g). The $^1$H-NMR spectrum is shown in Figure 9. HR-MS: calc. for $C_{19}H_{25}O_3[M+H^+]$ 301.1788, found 301.1798.

[Example L2: NORT]

The filtrate after removal of the white solid in Example 1 was concentrated under reduced pressure, and the obtained concentrate (144 g) was combined with the concentrate (231 g) of the fraction B obtained in Example 1. The mixture was distilled under reduced pressure at 220°C/0.6 to 2 hPa to obtain 168 g of a distillate. The obtained distillate was purified by silica gel column chromatography and dried under pressure at 50°C to obtain NORT (102 g). The $^1$H-NMR spectrum is shown in Figure 10. HR-MS: calc. for $C_{14}H_{19}O_3[M+H^+]$235.1324, found 235.1329.

[Example L3]

TCDT (0.75 g, 2.5 mmol) obtained in Example L1 and NORT (1.76 g, 7.5 mmol) obtained in Example L2 were mixed to obtain a mixture of TCDT:NORT = 25:75 (mol%).

[Example L4]

TCDT (1.50 g, 5.0 mmol) obtained in Example L1 and NORT (1.17 g, 5.0 mmol) obtained in Example L2 were mixed to obtain a mixture of TCDT:NORT = 50:50 (mol%).

[Example L5]

TCDT (2.25 g, 7.5 mmol) obtained in Example L1 and NORT (0.59 g, 2.5 mmol) obtained in Example L2 were mixed to obtain a mixture of TCDT:NORT = 75:25 (mol%).

[Example L6: Reduction of NORT]

NORT (4.78 g, 20.4 mmol) and chloro(1,5-cyclooctadiene)iridium(I) dimer (0.145 g, 0.216 mmol) were weighed into a 50 mL three-neck flask, and the flask was purged with nitrogen. Toluene (deoxidized grade manufactured by FUJIFILM Wako Pure Chemical Corp.; 10 mL) and 2-propanol (deoxidized grade manufactured by FUJIFILM Wako Pure Chemical Corp.; 10 mL) were weighed and added to the flask. Further, 1,2-bis(dicyclohexylphosphino)ethane (0.187 g, 0.442 mmol) was weighed and added to the flask. The flask was dipped in an oil bath of 100°C and heated for 6 hours with stirring using a magnetic stirrer. After the flask was allowed to cool, the contents were transferred to a 50 mL flask, and the solvent was distilled off using an evaporator. The concentrated residue was purified by silica gel column chromatography, and a fraction containing the compound of interest was recovered to obtain 5.01 g of a concentrate. This concentrate was dissolved in an ethyl acetate (2 mL)/heptane (10 mL) mixed solvent, and the solution was transferred to a freezer of -30°C and left standing overnight. A deposited solid was recovered by filtration under reduced pressure, then washed with heptane, and dried under reduced pressure at 60°C to obtain NORT-H (3.45 g). The $^1$H-NMR spectrum is shown in Figure 11. HR-MS: calc. for $C_{14}H_{21}O_3[M+H^+]$237. 1481, found 237.1485.

[Example L7: Reduction of TCDT]

TCDT-H (2.27 g) was obtained through the same reaction as in Example L2 except that TCDT (3.02 g) was used instead of NORT. The $^1$H-NMR spectrum is shown in Figure 12. HR-MS: calc. for $C_{19}H_{27}O_3[M+H^+]$303.1945, found 303.1955.

[Example L8]

NORT-H (0.59 g, 2.5 mmol) obtained in Example L6 and TCDT-H (2.27 g, 7.5 mmol) obtained in Example L7 were mixed to obtain a mixture of NORT-H/TCDT-H = 25:75 (mol%) .

[Example L9]

NORT-H (1.18 g, 5.0 mmol) obtained in Example L6 and TCDT-H (1.51 g, 5.0 mmol) obtained in Example L7 were mixed to obtain a mixture of NORT-H/TCDT-H = 50:50 (mol%) .

[Example L10]

NORT-H (1.77 g, 7.5 mmol) obtained in Example L6 and TCDT-H (0.75 g, 2.5 mmol) obtained in Example L7 were mixed to obtain a mixture of NORT-H/TCDT-H = 75:25 (mol%) .

[Example L11: $Me_5$NORT]

VCHC (8.63 g, 51.3 mmol) was weighed into a 50 mL three-neck flask, and the flask was purged with nitrogen. 1,2,3,4,5-Pentamethylcyclopentadiene (6.99 g, 51.3 mmol) was weighed and added to the flask. The flask was dipped in an oil bath of 180°C and heated for 14 hours with stirring using a magnetic stirrer. After the flask was allowed to cool, 1,2,3,4,5-pentamethylcyclopentadiene (6.55 g, 48.1 mmol) was added to the flask. The flask was dipped in an oil bath of 180°C and further heated for 20 hours. After the flask was allowed to cool, heptane (50 mL) was added to the flask, and the contents were rendered homogenous and left standing for a while, so that a solid was deposited. Therefore, the solution was transferred to a freezer of -30°C and left standing overnight. A deposited solid was recovered by filtration under reduced pressure, then washed with heptane (20 mL × twice), and dried under reduced pressure at 50°C to obtain $Me_5$NORT as a white solid (4.11 g). The $^1$H-NMR spectrum is shown in Figure 13. HR-MS: calc. for $C_{19}H_{29}O_3[M+H^+]$ 305.2105, found 305.2111.

[Usage Example L1]

T6C (1.05 g, 7.36 mmol) and a mixture of NORT:TCDT = 2:3 (2.01 g, 7.35 mmol) were weighed into a 50 mL three-neck flask, and the flask was purged with nitrogen. Dehydrated m-xylene (11.9 g) was added into the flask, and the mixture was then stirred using a magnetic stirrer for complete dissolution. The flask was dipped in a thermostat bath of 25°C. A tetrahydrofuran solution of potassium tert-butoxide (1.0 M, 28 μL, 0.28 mmol) was added thereto, and the mixture was stirred at 25°C for 1 hour. The reaction was terminated by the addition of acetic acid (0.010 g) (polymerization solution).

Subsequently, a reprecipitation operation was performed as described below for the evaluation of methanol-insoluble matter. The polymerization solution was diluted by the addition of acetone (61 g) and chloroform (31 g). The diluted solution was added into 400 g of methanol to deposit a polycarbonate resin. The deposited polycarbonate resin was recovered by filtration under reduced pressure and washed with methanol. The obtained polycarbonate resin was dried in vacuum at 100°C for 2 hours to obtain a polycarbonate resin (2.54 g). The glass transition temperature of the obtained polycarbonate resin was 169°C.

[Usage Example L2]

T6C (0.75 g, 5.28 mmol), a mixture of NORT-H:TCDT-H = 2:3 (1.46 g, 5.29 mmol), and dehydrated m-xylene (8.71 g) were weighed into a 25 mL three-neck flask and dehydrated using molecular sieves 4A (monomer solution). Another 25 mL three-neck flask was purged with nitrogen, and the monomer solution (10.04 g) was then collected into this flask. The flask was dipped in a thermostat bath of 25°C. A tetrahydrofuran solution of potassium tert-butoxide (1.0 M, 20 μL, 0.020 mmol) was added thereto, and the mixture was stirred at 25°C for 30 minutes. The reaction was terminated by the addition of acetic acid (0.011 g) (polymerization solution).

Subsequently, for the evaluation of methanol-insoluble matter, the polymerization solution was diluted by the addition of chloroform (39 g). The diluted solution was added into 284 g of methanol to deposit a polycarbonate resin. The

deposited polycarbonate resin was recovered by filtration under reduced pressure and washed with methanol. The obtained polycarbonate resin was dried in vacuum at 100°C for 2 hours to obtain a polycarbonate resin (1.87 g). The glass transition temperature of the obtained polycarbonate resin was 170°C.

[Usage Example L3]

NORT-H (3.01 g, 12.7 mmol) and dehydrated m-xylene (12.2 g) were weighed into a 50 mL three-neck flask and dehydrated using molecular sieves 4A (monomer solution). Another 50 mL three-neck flask was purged with nitrogen, and the monomer solution (11.96 g) was then collected into this flask. The flask was dipped in a thermostat bath of 25°C. A tetrahydrofuran solution of potassium tert-butoxide (1.0 M, 33 μL, 0.033 mmol) was added thereto, and the mixture was stirred at 25°C for 15 minutes. The reaction was terminated by the addition of acetic acid (0.010 g) (polymerization solution).

Subsequently, for the evaluation of methanol-insoluble matter, the polymerization solution was diluted by the addition of m-xylene (46 g). The diluted solution was added into 482 g of methanol to deposit a polycarbonate resin. The deposited polycarbonate resin was recovered by filtration under reduced pressure and washed with methanol. The obtained polycarbonate resin was dried in vacuum at 80°C for 4 hours to obtain a polycarbonate resin (2.21 g). The glass transition temperature of the obtained polycarbonate resin was 176°C.

[Usage Example L4]

T6C (0.32 g, 2.24 mmol), NORT-H (2.11 g, 8.93 mmol), and dehydrated m-xylene (9.81 g) were weighed into a 25 mL three-neck flask and dehydrated using molecular sieves 4A (monomer solution). Another 50 mL three-neck flask was purged with nitrogen, and the monomer solution (8.13 g) was then collected into this flask (the solution contained T6C: 0.21 g, NORT-H: 1.40 g, ad m-xylene: 6.52 g). The flask was dipped in a thermostat bath of 25°C. A tetrahydrofuran solution of potassium tert-butoxide (1.0 M, 4 μL, 0.004 mmol) was added thereto, and the mixture was stirred at 25°C for 30 minutes. The reaction was terminated by the addition of acetic acid (0.0040 g) (polymerization solution).

Subsequently, for the evaluation of methanol-insoluble matter, the polymerization solution was diluted by the addition of chloroform (22 g). The diluted solution was added into 287 g of methanol to deposit a polycarbonate resin. The deposited polycarbonate resin was recovered by filtration under reduced pressure and washed with methanol. The obtained polycarbonate resin was dried in vacuum at 100°C for 6 hours to obtain a polycarbonate resin (1.46 g). The glass transition temperature of the obtained polycarbonate resin was 166°C.

[Usage Example L5]

T6C (0.77 g, 5.40 mmol), TCDT-H (0.73 g, 2.42 mmol), and dehydrated m-xylene (12.50 g) were weighed into a 25 mL three-neck flask and dehydrated using molecular sieves 4A (monomer solution). Another 50 mL three-neck flask was purged with nitrogen, and the monomer solution (10.52 g) was then collected into this flask (the solution contained T6C: 0.58 g, TCDT-H: 0.55 g, and m-xylene: 9.39 g). The flask was dipped in a thermostat bath of 25°C. A tetrahydrofuran solution of potassium tert-butoxide (1.0 M, 5 μL, 0.005 mmol) was added thereto, and the mixture was stirred at 25°C for 30 minutes. The reaction was terminated by the addition of acetic acid (0.0022 g) (polymerization solution).

Subsequently, for the evaluation of methanol-insoluble matter, the polymerization solution was diluted by the addition of m-xylene (13 g). The diluted solution was added into 344 g of methanol to deposit a polycarbonate resin. The deposited polycarbonate resin was recovered by filtration under reduced pressure and washed with methanol. The obtained polycarbonate resin was dried in vacuum at 100°C for 6 hours to obtain a polycarbonate resin (0.97 g). The glass transition temperature of the obtained polycarbonate resin was 164°C.

[Usage Example L6]

T6C (3.69 g, 25.9 mmol), NORT-H (2.03 g, 8.59 mmol), TCDT-H (1.59 g, 5.26 mmol), and dehydrated m-xylene (29.30 g) were weighed into a 50 mL three-neck flask and dehydrated using molecular sieves 4A (monomer solution). Another 50 mL three-neck flask was purged with nitrogen, and the monomer solution (31.74 g) was then collected into this flask (the solution contained T6C: 3.19 g, NORT-H: 1.76 g, TCDT-H: 1.38 g, and m-xylene: 25.4 g). The flask was dipped in a thermostat bath of 25°C. A tetrahydrofuran solution of potassium tert-butoxide (1.0 M, 17 μL, 0.017 mmol) was added thereto, and the mixture was stirred at 25°C for 1 hour. The reaction was terminated by the addition of acetic acid (0.0061 g) (polymerization solution).

Subsequently, for the evaluation of methanol-insoluble matter, the polymerization solution was diluted by the addition of chloroform (119.56 g). The diluted solution was added into 730 g of methanol to deposit a polycarbonate resin. The deposited polycarbonate resin was recovered by filtration under reduced pressure and washed with methanol. The

obtained polycarbonate resin was dried in vacuum at 100°C for 4 hours to obtain a polycarbonate resin (5.42 g). The glass transition temperature of the obtained polycarbonate resin was 150°C.

[Usage Example L7]

T6C (4.81 g, 33.8 mmol), NORT-H (1.01 g, 4.27 mmol), TCDT-H (1.44 g, 4.76 mmol), and dehydrated m-xylene (29.04 g) were weighed into a 50 mL three-neck flask and dehydrated using molecular sieves 4A (monomer solution). Another 50 mL three-neck flask was purged with nitrogen, and the monomer solution (32.06 g) was then collected into this flask (the solution contained T6C: 4.25 g, NORT-H: 0.89 g, TCDT-H: 1.28 g, and m-xylene: 25.6 g). The flask was dipped in a thermostat bath of 25°C. A tetrahydrofuran solution of potassium tert-butoxide (1.0 M, 19 μL, 0.019 mmol) was added thereto, and the mixture was stirred at 25°C for 1 hour. The reaction was terminated by the addition of acetic acid (0.0062 g) (polymerization solution).

Subsequently, for the evaluation of methanol-insoluble matter, the polymerization solution was diluted by the addition of chloroform (116.26 g). The diluted solution was added into 718 g of methanol to deposit a polycarbonate resin. The deposited polycarbonate resin was recovered by filtration under reduced pressure and washed with methanol. The obtained polycarbonate resin was dried in vacuum at 100°C for 4 hours to obtain a polycarbonate resin (5.33 g). The glass transition temperature of the obtained polycarbonate resin was 144°C.

[Usage Example L8]

T6C (3.99 g, 28.1 mmol), TCDT-H (2.72 g, 9.00 mmol), and dehydrated m-xylene (26.93 g) were weighed into a 50 mL three-neck flask and dehydrated using molecular sieves 4A (monomer solution). Another 50 mL three-neck flask was purged with nitrogen, and the monomer solution (29.79 g) was then collected into this flask (the solution contained T6C: 3.54 g, TCDT-H: 2.41 g, and m-xylene: 23.8 g). The flask was dipped in a thermostat bath of 25°C. A tetrahydrofuran solution of potassium tert-butoxide (1.0 M, 16 μL, 0.016 mmol) was added thereto, and the mixture was stirred at 25°C for 1 hour. The reaction was terminated by the addition of acetic acid (0.0053 g) (polymerization solution).

Subsequently, for the evaluation of methanol-insoluble matter, the polymerization solution was diluted by the addition of chloroform (110 g). The diluted solution was added into 799 g of methanol to deposit a polycarbonate resin. The deposited polycarbonate resin was recovered by filtration under reduced pressure and washed with methanol. The obtained polycarbonate resin was dried in vacuum at 100°C for 4 hours to obtain a polycarbonate resin (5.30 g). The glass transition temperature of the obtained polycarbonate resin was 149°C.

[Comparative Usage Example L1]

A polycarbonate resin was obtained by polymerization reaction and a reprecipitation operation by the same methods as in Usage Example L3 except that T6C was used instead of NORT-H. The glass transition temperature of the obtained polycarbonate resin was 120°C.

Industrial Applicability

The polycarbonate resin, the polycarbonate resin composition, and an optical molded article comprising the same according to the present invention have industrial applicability in the fields of, for example, various optical materials such as optical lens materials, optical devices, materials for optical components, and display materials.

## Claims

**1.** A polycarbonate resin having a structural unit represented by the following formula (1):

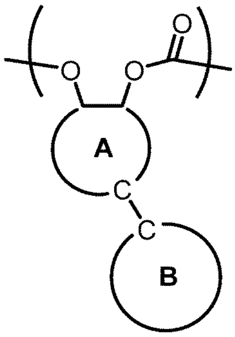

(1)

wherein A is an optionally substituted trivalent alicyclic moiety, and B is an optionally substituted monovalent alicyclic moiety.

**2.** The polycarbonate resin according to claim 1,
wherein the structural unit represented by the formula (1) has a structural unit represented by the following formula (1A):

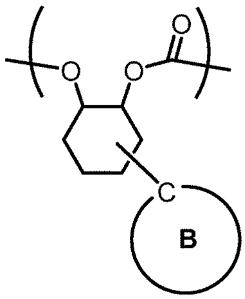

(1A)

wherein B is an optionally substituted monovalent alicyclic moiety.

**3.** The polycarbonate resin according to claim 1,
wherein the alicyclic moiety B is a group represented by the following formula (1a):

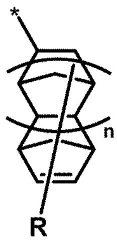

(1a)

wherein R is a substituent, n is an integer of 0 to 2, and * is a binding site.

**4.** The polycarbonate resin according to claim 1,
wherein the alicyclic moiety B is a group represented by the following formula (1b):

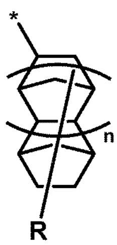

(1b)

wherein R is a substituent, n is an integer of 0 to 2, and * is a binding site.

5. The polycarbonate resin according to claim 1,
wherein the polycarbonate resin has a structural unit represented by the following formula (1B):

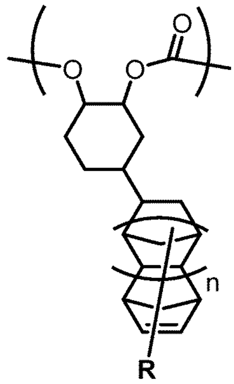
(1 B)

wherein R is a substituent, and n is an integer of 0 to 2.

6. The polycarbonate resin according to claim 1,
wherein the polycarbonate resin has a structural unit represented by the following formula (1C):

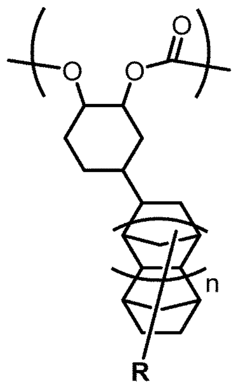
(1 C)

wherein R is a substituent, and n is an integer of 0 to 2.

7. The polycarbonate resin according to claim 1,
wherein the polycarbonate resin has a structural unit represented by the following formula (1B-H):

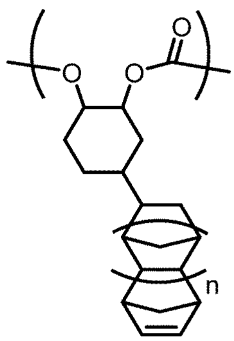
(1 B－H)

wherein n is an integer of 0 to 1.

8. The polycarbonate resin according to claim 1,

wherein the polycarbonate resin has a structural unit represented by the following formula (1C-H):

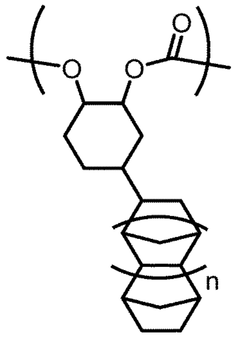

(1 C−H)

wherein n is an integer of 0 to 1.

**9.** The polycarbonate resin according to claim 1,
wherein the polycarbonate resin has a structural unit represented by the following formula (2):

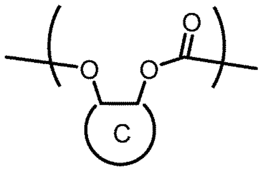

(2)

wherein C is an optionally substituted divalent alicyclic moiety.

**10.** The polycarbonate resin according to claim 1,
wherein a weight-average molecular weight (Mw) is 10,000 or larger and 1,000,000 or smaller.

**11.** The polycarbonate resin according to claim 1,
wherein a glass transition temperature (Tg) is 125°C or higher and 250°C or lower.

**12.** The polycarbonate resin according to claim 1,
wherein an absolute value of a photoelastic coefficient is $1.5 \times 10^{-12}$ $Pa^{-1}$ or smaller.

**13.** The polycarbonate resin according to claim 1,
wherein an absolute value of an in-plane phase difference in a 100% uniaxially stretched film of the polycarbonate resin is 100 nm or smaller in terms of a thickness of 100 μm.

**14.** The polycarbonate resin according to claim 1,
wherein temperature dependence of orientation double refraction (dΔn/dT) in a stretched film obtained by uniaxial stretching under the following conditions satisfies $-0.1 \times 10^{-5} \leq d\Delta n/dT \leq +0.1 \times 10^{-5}$:

(stretching conditions)
stretching temperature: a temperature higher by 20°C than glass transition temperature Tg of the polycarbonate resin
stretching rate: 50 mm/min
stretch ratio: 100%

**15.** A polycarbonate resin composition comprising the polycarbonate resin according to any one of claims 1 to 14 and an antioxidant.

**16.** An optical component comprising the polycarbonate resin according to any one of claims 1 to 14.

**17.** Use of the polycarbonate resin according to any one of claims 1 to 14 as a material for an optical component.

**18.** A method for producing a polycarbonate resin having a structural unit represented by the following formula (1), comprising
a polymerization step of ring-opening polymerizing a cyclic carbonate represented by the following formula (1L) to obtain the polycarbonate resin:

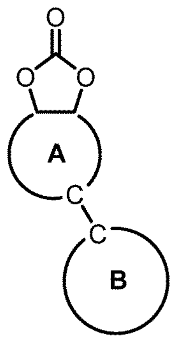

(1 L)

wherein A is an optionally substituted trivalent alicyclic moiety, and B is an optionally substituted monovalent alicyclic moiety,

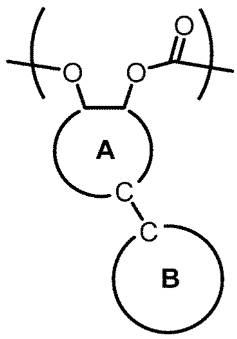

(1)

wherein A is an optionally substituted trivalent alicyclic moiety, and B is an optionally substituted monovalent alicyclic moiety.

**19.** A cyclic carbonate having a structure where an alicyclic skeleton is bonded to a 1,2-cycloalkylene carbonate skeleton via a single bond.

**20.** The cyclic carbonate according to claim 19, wherein the cyclic carbonate is represented by the following formula (1L):

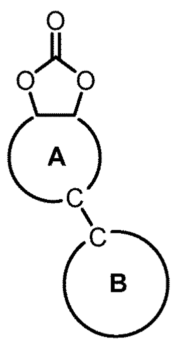

(1 L)

wherein A is an optionally substituted trivalent alicyclic moiety, and B is an optionally substituted monovalent alicyclic moiety.

**21.** The cyclic carbonate according to claim 20, wherein the formula (1L) is the following formula (1LA):

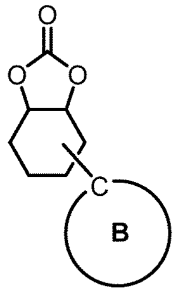

(1LA)

wherein B is an optionally substituted monovalent alicyclic moiety.

**22.** The cyclic carbonate according to claim 20, wherein the alicyclic moiety B is a group represented by the following formula (1a):

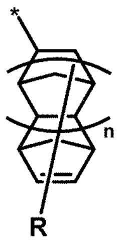

(1a)

wherein R is a substituent, n is an integer of 0 to 2, and * is a binding site.

**23.** The cyclic carbonate according to claim 20, wherein the alicyclic moiety B is a group represented by the following formula (1b):

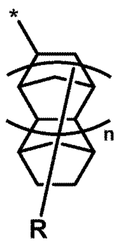

(1b)

wherein R is a substituent, n is an integer of 0 to 2, and * is a binding site.

**24.** The cyclic carbonate according to claim 20, wherein the alicyclic moiety B is an optionally substituted norbornyl group, an optionally substituted norbornenyl group, an optionally substituted decahydro-1,4:5,8-dimethanonaphthalenyl group, or an optionally substituted octahydro-1,4:5,8-dimethanonaphthalenyl group.

**25.** The cyclic carbonate according to claim 19, wherein the cyclic carbonate is represented by the following formula (1LB):

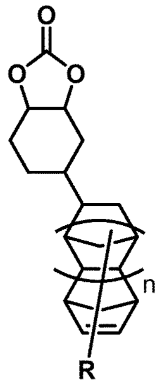

(1 L B)

wherein R is a substituent, and n is an integer of 0 to 2.

**26.** The cyclic carbonate according to claim 19, wherein the cyclic carbonate is represented by the following formula (1LC):

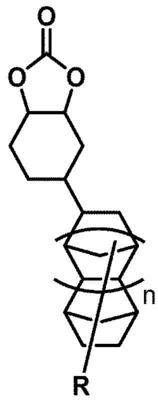

(1 L C)

wherein R is a substituent, and n is an integer of 0 to 2.

**27.** The cyclic carbonate according to claim 19, wherein the cyclic carbonate is represented by

the following formula (1L-1):

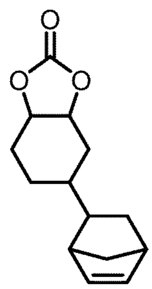

(1 L − 1)

the following formula (1L-2):

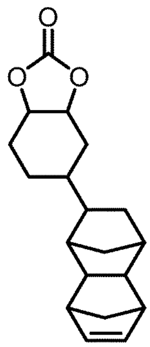

(1L-2)

the following formula (1L-3):

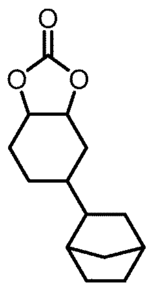

(1L-3)

or the following formula (1L-4):

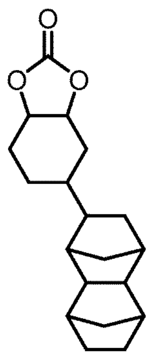

(1L-4)

**28.** A method for producing a cyclic carbonate having a structure where an alicyclic skeleton is bonded to a 1,2-cycloalkylene carbonate skeleton via a single bond, comprising the step of reacting a cyclic carbonate represented by the following formula (2L) with a compound having a conjugated diene moiety:

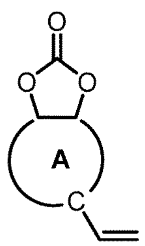

(2L)

wherein A is an optionally substituted trivalent alicyclic moiety.

**29.** A cyclic carbonate composition comprising

a cyclic carbonate represented by the following formula (1L-1):

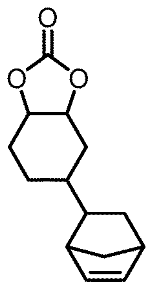

(1 L－1)

and a cyclic carbonate represented by the following formula (1L-2):

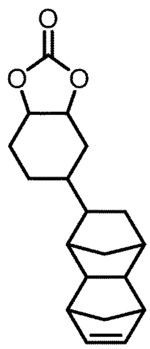

(1 L－2)

**30.** A cyclic carbonate composition comprising

a cyclic carbonate represented by the following formula (1L-3):

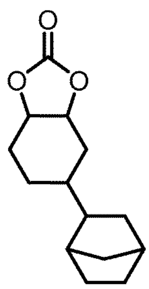

(1 L－3)

and a cyclic carbonate represented by the following formula (1L-4):

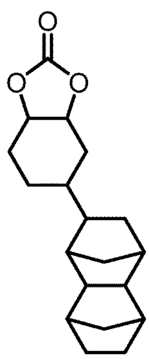

(1 L－4)

Figure 1

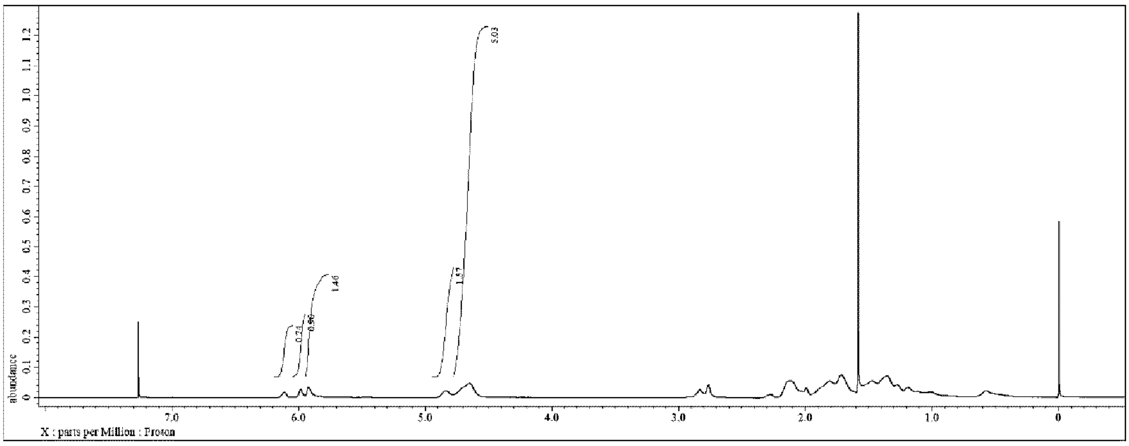


Figure 2

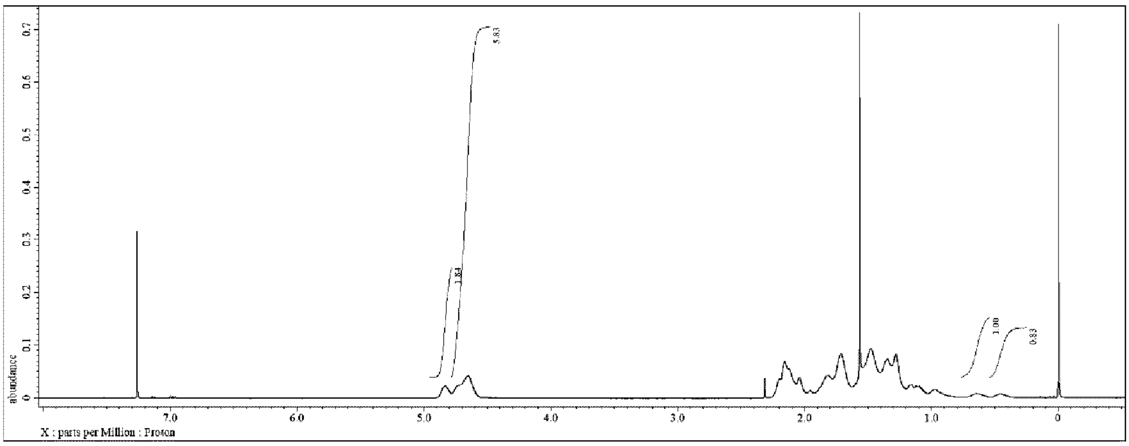

Figure 3

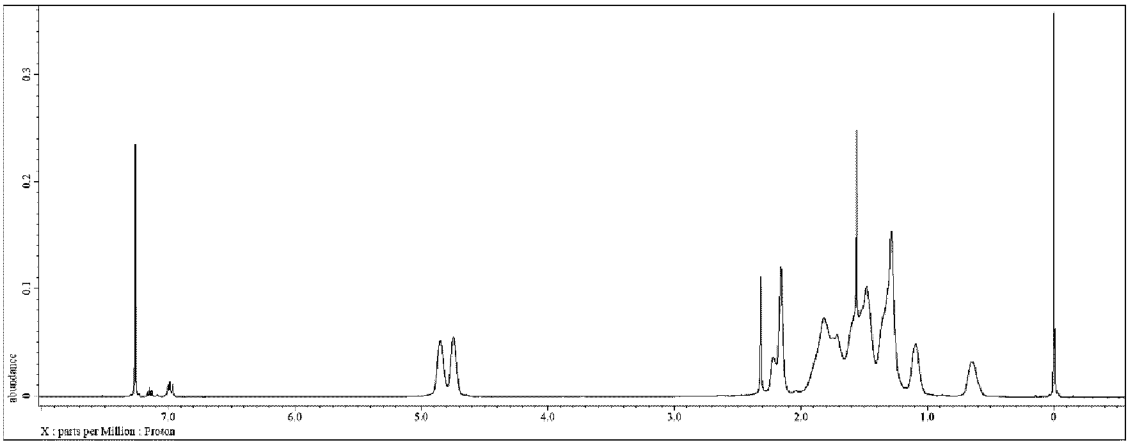


Figure 4

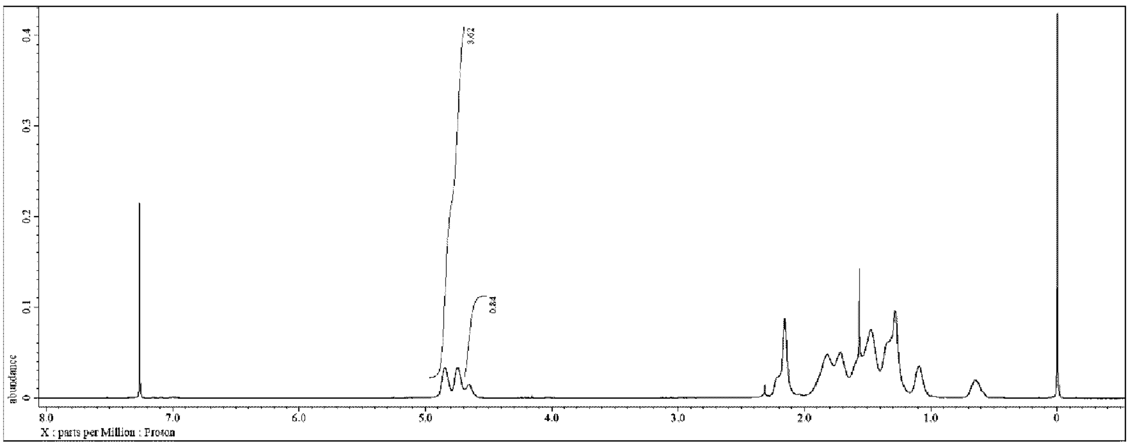

Figure 5

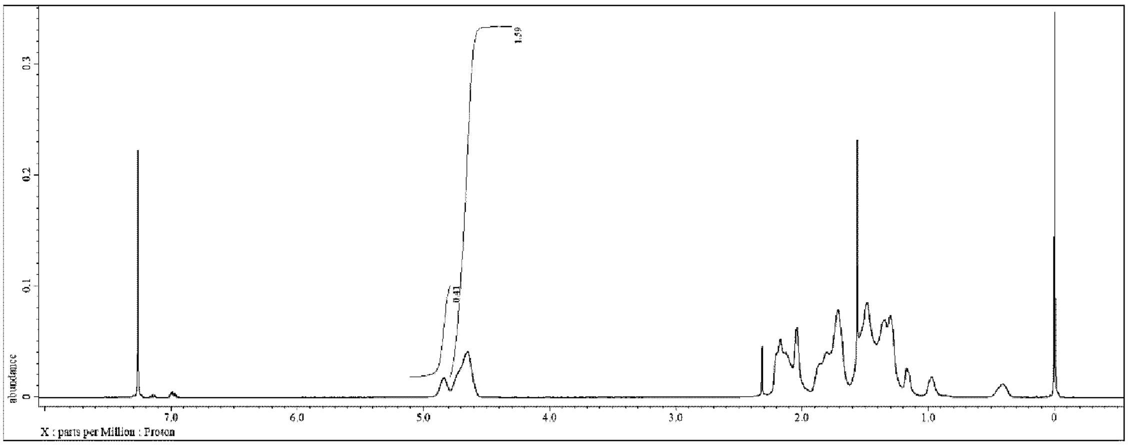


Figure 6

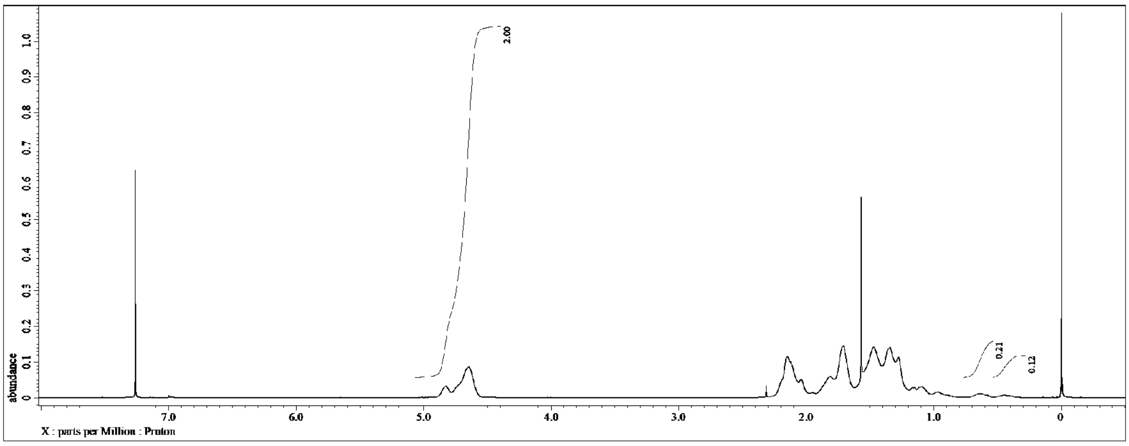


Figure 7

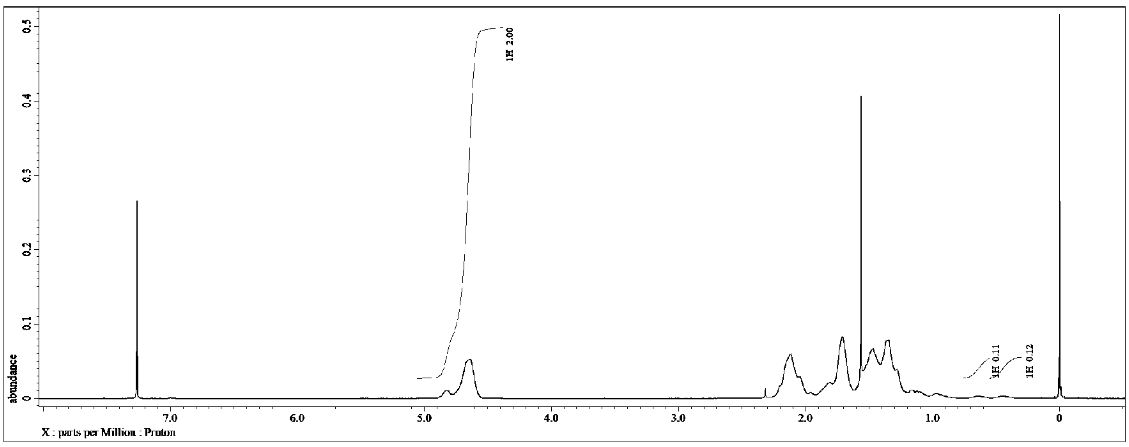

Figure 8

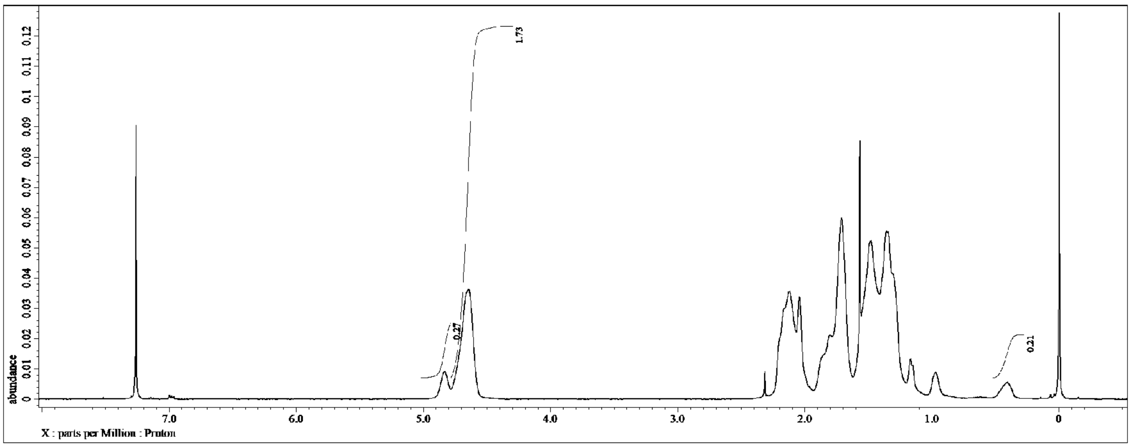


Figure 9

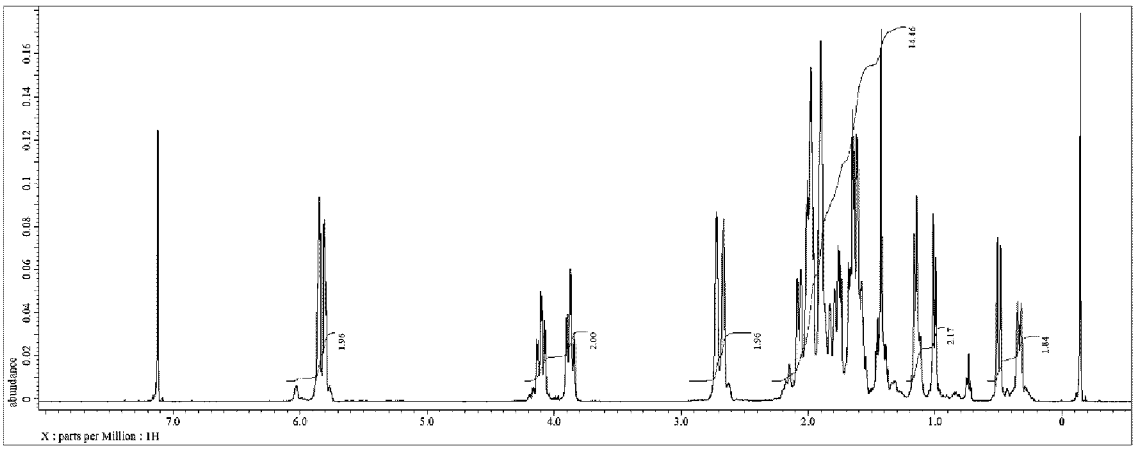

Figure 10

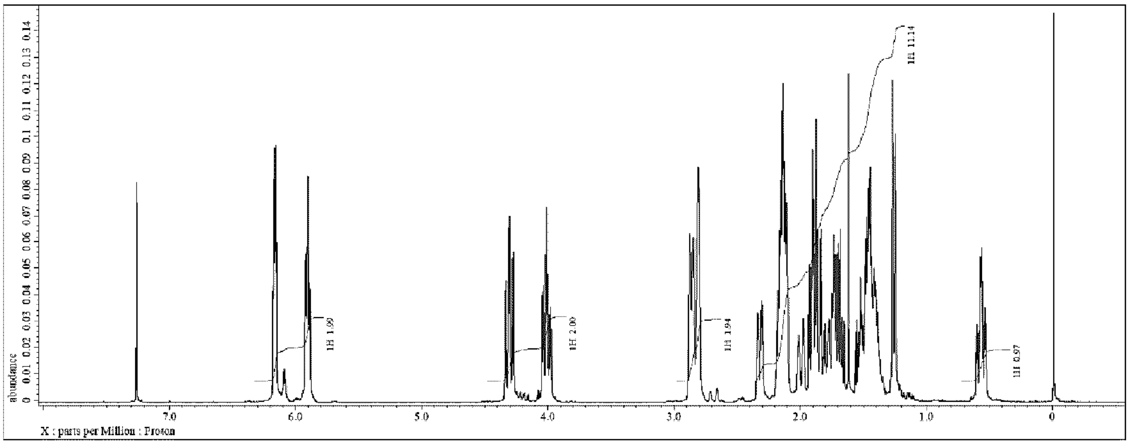


Figure 11

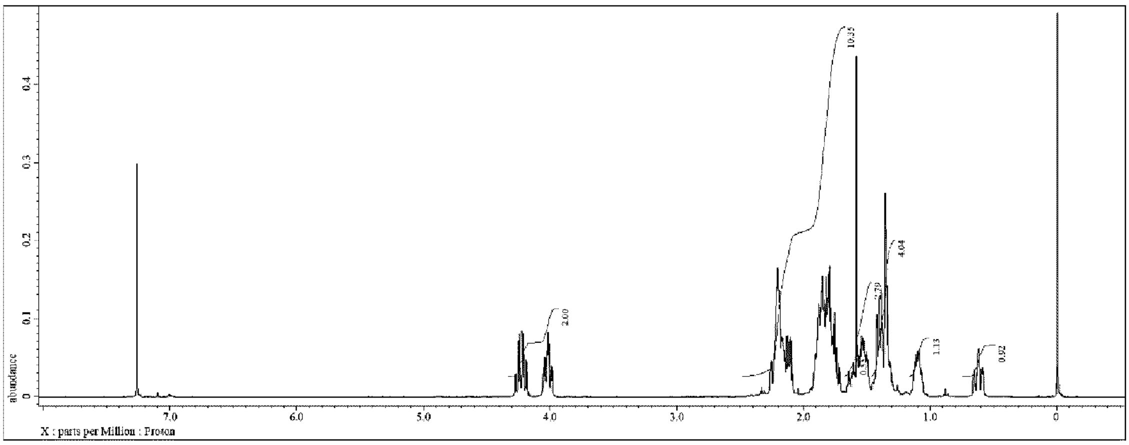

Figure 12

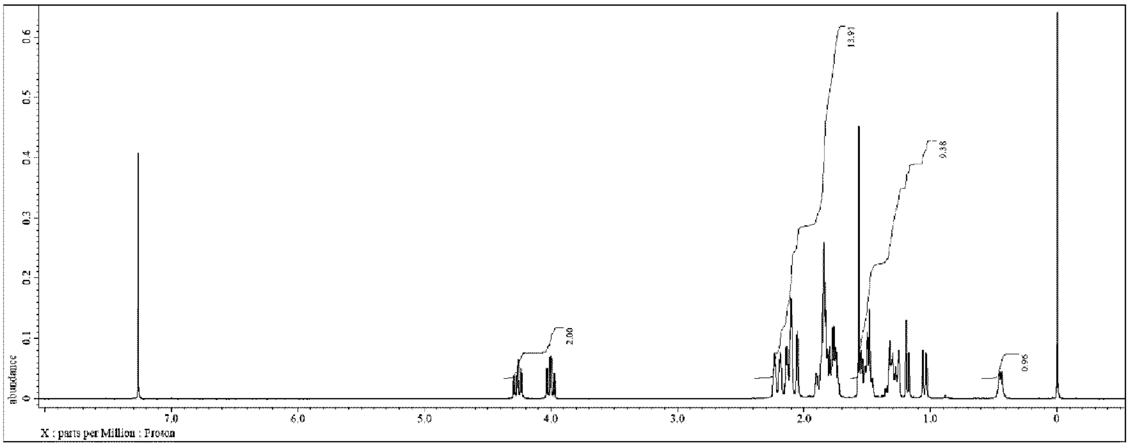


Figure 13

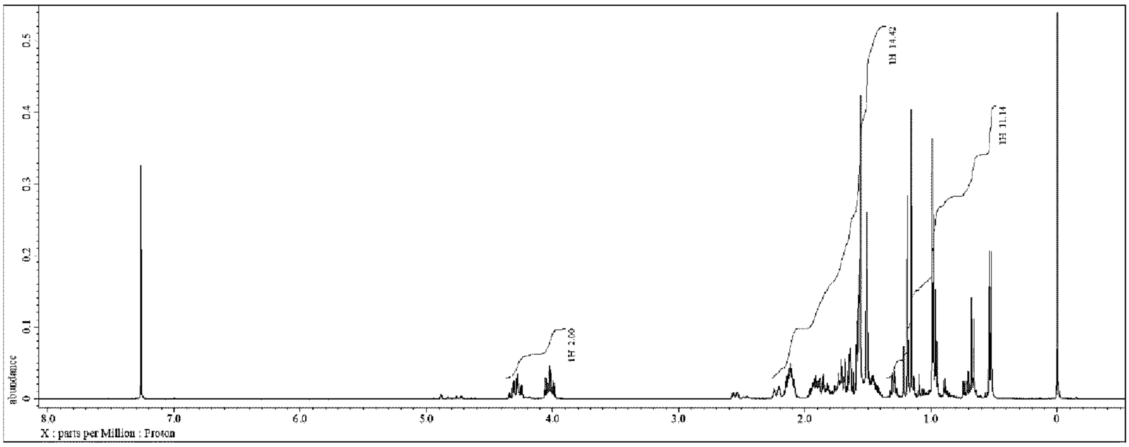

## INTERNATIONAL SEARCH REPORT

International application No.
**PCT/JP2023/011315**

**A. CLASSIFICATION OF SUBJECT MATTER**

***C08G 64/02***(2006.01)i; ***C07D 317/44***(2006.01)i; ***C07D 317/46***(2006.01)i; ***C08G 64/30***(2006.01)i; ***C08G 64/38***(2006.01)i; ***C08L 69/00***(2006.01)i; ***G02B 1/04***(2006.01)i
FI: C08G64/02; C07D317/44; C07D317/46 CSP; C08G64/30; C08G64/38; C08L69/00; G02B1/04

According to International Patent Classification (IPC) or to both national classification and IPC

**B. FIELDS SEARCHED**

Minimum documentation searched (classification system followed by classification symbols)

C08G64/02; C07D317/44; C07D317/46; C08G64/30; C08G64/38; C08L69/00; G02B1/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | CN 107991842 A (ZHEJIANG UNIVERSITY) 04 May 2018 (2018-05-04)<br>entire text | 1-30 |
| A | WO 2021/145443 A1 (ASAHI KASEI KABUSHIKI KAISHA) 22 July 2021 (2021-07-22)<br>entire text | 1-30 |
| A | WO 2007/020876 A1 (UBE INDUSTRIES, LIMITED) 22 February 2007 (2007-02-22)<br>entire text | 1-30 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 May 2023** | **13 June 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/011315**

| Patent document cited in search report | Publication date (day/month/year) | Patent family member(s) | Publication date (day/month/year) |
|---|---|---|---|
| CN 107991842 A | 04 May 2018 | (Family: none) | |
| WO 2021/145443 A1 | 22 July 2021 | EP 4092068 A1<br>entire text<br>CN 114945619 A | |
| WO 2007/020876 A1 | 22 February 2007 | US 2009/0170006 A1<br>entire text<br>EP 1916734 A1<br>CN 101243575 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4774610 B
- JP 6507495 B
- JP 5403537 B
- JP 2019108547 A


### Non-patent literature cited in the description


- *Macromolecules*, 2014, vol. 47, 4230-4235
- *Polymer Engineering and Science*, 1999, vol. 39, 2349-2357